# EUROPEAN PATENT SPECIFICATION

(11) **EP 1 837 993 B1**
(45) Date of publication and mention of the grant of the patent: **27.01.2010**
(21) Application number: 06111477.3
(22) Date of filing: 21.03.2006
(51) Int. Cl.: H03K 17/10, G11C 16/12, H01L 27/02

(54) **Improvements to the design of voltage switches**
Verbesserungen am Entwurf von Spannungsschalter
Améliorations dans la conception de commutateurs de tension

(43) Date of publication of application: 26.09.2007
(73) Proprietor: STMicroelectronics S.r.l., 20041 Agrate Brianza (Milano) (IT)
(72) Inventor: Campardo, Giovanni, 24128 Bergamo (BG) (IT); Micheloni, Rino, 22078 Turate (CO) (IT)
(74) Representative: W.P. Thompson & Co.

(56) References cited:
- US-A1- 2001 020 840

## Description

The present invention relates to the field of the semiconductor Integrated Circuits (ICs), and more particularly, it relates to ICs that have to manage differentiated voltage levels.

The European Patent Office cited as the closest prior art during prosecution the US patent application filed in the name of Kojima, assignee Matsushita Electric Industrial Co. Ltd., published under US 2001/0020840. This describes a circuit implemented within a flash memory intended to enable a wordline driver of low-level voltage type to handle not only low voltages but also high-level voltages.

ICs can be classified in two broad categories, depending on the entity of the voltage differences that occur across the terminals of the electronic devices included therein.

More specifically, in ICs belonging to a first category, called from now on "low voltage ICs", all the electronic devices included therein (e.g., the transistors and the capacitors) are designed in such a way to guarantee the capability of sustaining, between their terminals, voltage differences that are up-limited (in absolute value) by a predetermined maximum voltage difference ΔVml, e.g. equal to the IC supply voltage value Vdd. The voltage differences experienced by the devices included in the low voltage ICs are such as to allow the correct functioning of standard devices (like those making up the logic circuitry included in ICs) without causing malfunctioning or breaking thereof, that may be caused instead by excessive voltage difference values. Referring for example to ICs including transistors like BJTs or FETs, particularly IGFETs like MOS (Metal Oxide Semiconductor) FETs, the predetermined maximum voltage difference ΔVml may be equal to the supply voltage Vdd, in such a way to avoid breaking of gate insulators (oxides), or undesired junctions breakdown. In particular, as far as MOSFETs are concerned, Low Voltage (LV) MOS transistors (hereinafter, LV transistors) are devices adapted to guarantee the capability of sustaining between their terminals voltage differences that are up-limited by the IC supply voltages Vdd.

A low voltage IC is designed in such a way that, in operation, practically all the transistors included therein experience voltage differences equal to the supply voltage Vdd or less, at least between their control terminal, e.g., the gate terminal, and any other terminal thereof (thus avoiding breaking of gate insulators), and preferably between pair of terminals corresponding to semiconductor junctions that may be susceptible of breakdown.

The ICs belonging to the second category, called from now on "high voltage ICs", include instead, in addition to low-voltage devices, devices designed in such a way to guarantee the capability of sustaining, at least between a pair of their terminals, voltage differences up-limited (in absolute value) by a predetermined maximum voltage difference ΔVmh higher than the predetermined maximum voltage difference ΔVml that the devices included in low voltage ICs are capable of sustaining. Referring again to the example of circuits including transistors, the high voltage ICs include one or more transistors that, in operation, may happen to be biased in such a way to experience voltage differences higher than the IC supply voltage Vdd at least between their control terminal and one or more other terminals thereof.

High voltage ICs are for example common in the field of memory circuits. In particular, in non-volatile memories high voltages are needed to modify the stored data (e.g., to program and/or erase the memory cells of the memory, in such a way to activate known physical phenomena such as Channel Hot Electron - CHE - injection and Fowler-Nordheim Tunneling - FNT). The transistors used in such high voltage ICs need to be capable of withstanding high voltage differences across their terminals, without damaging or malfunctioning, for example not to cause gate insulators breaking or junction breakdown, or not to trigger CHE or FNT (in fact, while for a memory cell the CHE and FNT effects are desired and controlled for the purposes of modifying the stored data, said effects are in turn deleterious for the transistors of the rest of the IC, like the logic circuitry).

As another example, in volatile memories, such as in Static RAMs (SRAMs) and Dynamic RAMs (DRAMs), high voltages higher than the IC supply voltage Vdd can be used for biasing the cells of the memory, in such a way to improve the speed of the reading operations.

In the case of high voltage ICs comprising MOS transistors, High Voltage (HV) MOS transistors (hereinafter, HV transistors) need to be designed, engineered and integrated (with ad-hoc manufacturing process steps), which are adapted to guarantee the capability of sustaining, at least between a pair of their terminals, voltages difference up-limited by the predetermined maximum voltage difference ΔVmh, higher than the IC supply voltage Vdd. In particular, HV transistors may have a gate insulator layer thicker than that normally used for the standard, LV transistors, i.e., the MOS transistors typically used in low voltage ICs. HV transistors thus have a higher threshold voltage value, and the use of a thicker gate insulator avoids the insulator breaking, even with relatively high voltage differences applied between the gate and, e.g., the channel region.

However, the necessity of using HV transistors poses constraints to the technology used to fabricate the IC. More particularly, even if the scaling of the transistors size, made possible by the evolution of the manufacturing technologies, allows to drastically reduce the dimensions of the LV transistors, the gate insulator thickness of the HV transistors can not be thinned, not to jeopardize the capability of sustaining the desired voltages. Consequently, it is not possible to shrink the dimensions of the HV transistors, and thus it is hard to achieve the desired reduction in the silicon area occupied by the ICs including high voltage circuits.

One of the methods that is more commonly adopted, is to design high voltage ICs using both LV and HV transistors. More particularly, since the HV transistors occupy more silicon area compared to the LV transistors, it is possible to save silicon area using HV transistors only where they are strictly necessary. However, this method has a major drawback, given by the fact that the manufacturing process has to include a higher number of processing steps and masks, for example for differentiating the insulator thickness and the threshold voltages of the HV and LV transistors.

A structure that is often used in high voltage ICs is the so-called "voltage switch", which is a circuit able to selectively connect one selected input terminal, selected among two or more input terminals each one adapted to supply a respective voltage, to a switch output terminal, while keeping the latter electrically insulated from the unselected input terminals. The voltage differences between said input terminals may be higher than the IC supply voltage. Consequently, voltage switches of such type usually have to include HV transistors, posing the constraints to the IC manufacturing technology that have been previously described.

The use of HV transistors for realizing voltage switches is usually strongly requested when some of the functional blocks of an IC need for their operation both positive voltages higher than the IC supply voltage and negative voltages lower than the most negative supply voltage (typically the ground), as in the case of flash memory ICs. In this case, in fact, a control terminal of a selected generic memory cell may have to be biased to a voltage value much higher than the IC supply voltage during a program operation, while during an erase operation (of the memory sector to which the cell belongs) a negative control terminal voltage value may be required. For this purpose, an IC may need to include boosting circuits adapted to generate the required high voltages (both positive and negative) starting from the IC supply voltage. In particular, the IC has to comprise both a negative charge pump and a positive charge pump. Since it may be necessary to bias a same circuital node (e.g., the control terminal of a generic memory cell) with either negative or positive voltage values, depending on the operation to be conducted, said node may require to be selectively connected either to the output terminal of the positive charge pump or to the output terminal of the negative charge pump, in such a way to be able to receive the required voltage values. For this purpose, a voltage switch is used for selectively connecting the output terminal of the positive charge pump or the output terminal of the negative charge pump to an output common node, from which the voltage value received from one or the other of the charge pumps is distributed through the IC to the predetermined circuit structures. The voltage switch is usually realized using HV transistors, because, when one of the two charge pumps is on, and its voltage is to be transferred to the switch output terminal, a voltage difference between the switch output terminal and the output terminal of the other charge pump may be higher (in absolute value) than the IC supply voltage.

In view of the state of the art outlined in the foregoing, the Applicant has faced the problem of how to improve the known solutions for implementing voltage switches. Particularly, the Applicant has tackled the problem of how to realize a voltage switch that does not make use of HV transistors.

The Applicant has found that, by adopting a particular circuit topology for realizing the voltage switches, it is possible to avoid using HV MOS transistors, with great benefits in terms, for example, of reduced silicon area and simplification of the manufacturing process.

According to an aspect of the present invention, there is a circuit comprising:
a first input terminal (INA) to provide a first high voltage n times larger than a supply voltage Vdd when a second input terminal (INB) assumes a reference level, the second input to provide a second high voltage m times larger than Vdd when the first input terminal assumes the reference level, wherein the absolute value of at least one of n and m is greater than unity;
an output terminal (OUT);
the circuit being characterised in that it comprises:
a first plurality of low voltage transistors coupled in series from the first input terminal to the output terminal and a second plurality of low voltage transistors coupled in series from the second input terminal to the output terminal, each low voltage transistor of the first and second plurality rated to sustain a maximum voltage difference between terminals that is below at least one of the first and second input voltages;
a biasing means coupled to a control terminal of each low voltage transistor of the first and second pluralities, wherein the biasing means is configured to alternately couple the output terminal with one of the first and second input terminals while electrically isolating the other input terminal by biasing each of the control terminals at levels to switch ones of the first and second plurality between a pass state and a reversed diode state without exceeding the maximum voltage difference rating of any low voltage transistor of the first and second pluralities.

The characterizing features of the present invention are set forth in the appended claims. The invention itself, however, as well as further features and advantages thereof will be best understood by reference to the following detailed description, given purely by way of a non-restrictive example, to be read in conjunction with the accompanying drawings:
Figure 1 is an electronic circuit schematic of a voltage switch adapted to receive a first voltage of 6 Volts and a second voltage of -3 Volts, according to an embodiment of the present invention;
Figure 2 is an electronic circuit schematic of a voltage switch adapted to receive a first voltage of 9 Volts and a second voltage of -3 Volts, according to an embodiment of the present invention;
Figure 3 is an electronic circuit schematic of a voltage switch adapted to receive a first voltage of 6 Volts and a second voltage of -6 Volts, according to an embodiment of the present invention;
Figure 4 is an electronic circuit schematic of a voltage switch adapted to receive a first voltage of 9 Volts and a second voltage of -6 Volts, according to an embodiment of the present invention;
Figure 5 is an electronic circuit schematic of a voltage switch adapted to receive a first voltage of 12 Volts and a second voltage of -9 Volts, according to an embodiment of the present invention; and
Figure 6 is an electronic circuit schematic of a generic two-inputs voltage switch capable of receiving a generic pair of voltage values at its inputs, according to an embodiment of the present invention.

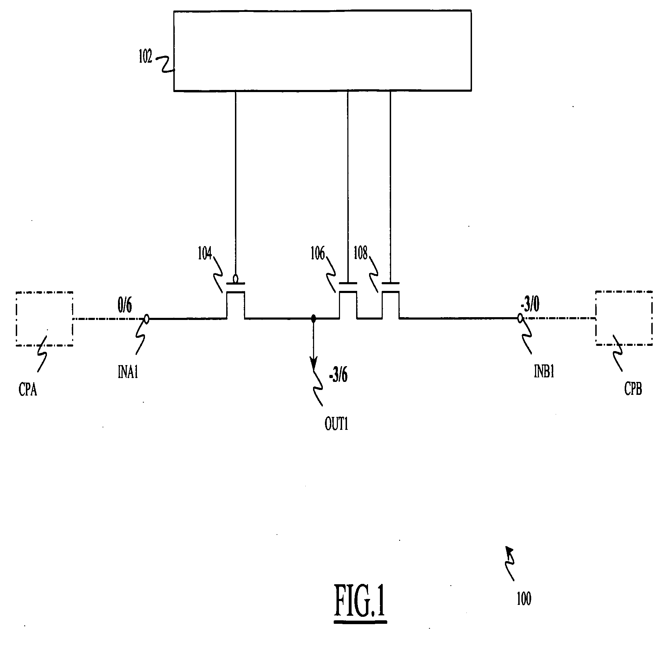

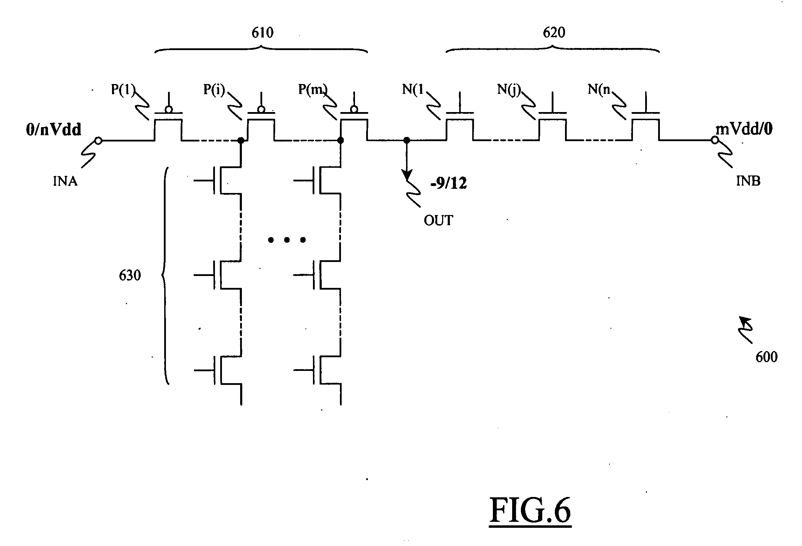

In the following of this document, making reference to Figures 1 to 5 various examples of voltage switches will be described according to possible embodiments of the present invention; in particular, different structures are presented which depend on the voltage values provided at their input terminals. After the illustration of said exemplary voltage switches, a more general scheme will be provided making reference to Figure 6 for designing a generic two-inputs voltage switch capable of receiving a generic pair of voltage values at its inputs, that make use of LV transistors only.

In all the cases illustrated in the following, merely by way of example, it is assumed to use as LV transistors MOS transistors, both p-channel (pMOS) and n-channel (nMOS) transistors, having a threshold voltage of 1 Volt (in absolute value). Moreover, it is assumed to use a supply IC voltage Vdd that is equal to 3 Volts with respect to a reference voltage (ground voltage, GND). The values of all the voltages that will be taken into account in this description are to be intended as calculated with respect to the value of the ground voltage GND.

Figure 1 illustrates the electronic circuit schematic of a voltage switch 100 according to an embodiment of the invention having a first input terminal INA1 for receiving a first voltage of. For example, 6 Volts and a second input terminal INB1 for receiving a second voltage of, for example, -3 Volts. The voltage switch 100 further includes an output terminal OUT1 adapted to be selectively coupled with the first input terminal INA1 or with the second input terminal INB1, so as to receive the voltages present thereat, and a biasing control block 102, adapted to bias the MOS transistors included in the voltage switch 100.

The input terminals INA1 and INB1 are assumed to be connected to respective circuital nodes, each of which has a voltage that is in turn assumed to vary between two values; for example, the first input terminal INA1 may be connected to a circuital node whose voltage varies from 6V and the ground voltage GND, whereas the second input terminal INB 1 may be connected to a circuital node whose voltage varies from -3Volts to the ground voltage GND. In particular, when the first input terminal INA1 receives, from the respective node, the first voltage of 6 Volts, and is coupled to the output terminal OUT1 by the voltage switch 100, the other input terminal INB 1 receives from the respective node the ground voltage GND, and is kept isolated from the output terminal OUT1. When instead the second input terminal INB1 receives from the respective node the second voltage of -3 Volts, and is coupled to the output terminal OUT1 by the voltage switch 100, the other input terminal INA1 receives from the respective node the ground voltage GND, and is kept isolated from the switch output terminal OUT1. In particular, the two input terminals INA1 and INB1 may be connected to output nodes of respective charge pumps CPA and CPB. Alternatively, the voltages may be received from outside the IC.

The voltage switch 100 includes, on the side of the first input terminal INA1, a pMOS transistor 104 having a first terminal connected to the first input terminal INA1, a second terminal connected to the output terminal OUT1 and a control terminal connected to the biasing control block 102. The voltage switch 100 further includes, on the side of the second input terminal INB1, an nMOS transistor 106 having a first terminal connected to the output terminal OUT1, a second terminal connected to a first terminal of a further nMOS transistor 108 and a control terminal connected to the biasing control block 102. The nMOS transistor 108 has a control terminal connected to the biasing control block 102 and a second terminal connected to the second input terminal INB 1.

When it is desired to provide the first voltage of 6 Volts present at the first input terminal INA1 to the output terminal OUT1, the first input terminal INA1 is coupled to the output terminal OUT1, while the second input terminal INB1 (in this case, assumed to be biased to the ground voltage GND) has to be electrically kept insulated therefrom. Thus, the circuital branch on the side of the first input terminal INA1 (in this case, formed by the pMOS transistor 104) has to be capable of setting up a conductive, low resistance path from the input terminal INA1 to the output terminal OUT1, in such a way to transfer the voltage of 6 Volts thereto. Thus, the pMOS transistor 104 has the function of conveying the voltage of 6 Volts from its first terminal to its second terminal; in other words, the pMOS transistor 104 acts as a pass transistor. A p-channel MOS transistor instead of an n-channel may be preferred, since pMOS pass transistors are able to convey positive voltages better than nMOS pass transistors.

For activating the pMOS transistor 104, the biasing control block 102 biases the transistor control terminal to a voltage lower than the voltage (in this case, 6 Volts) that has to be conveyed from the first to the second transistor terminal by an amount of at least the pMOS transistor threshold voltage (in the present example, at least 1 Volt). For example, the biasing control block 102 biases the control terminal of the pMOS transistor 104 to a voltage equal to 3 Volts. In this way, when it is activated, the pMOS transistor 104 has voltage differences across its control terminal and the other terminals thereof that are at most equal to the value of the supply voltage Vdd, and this allows using an LV transistor for the pMOS transistor 104. Lower voltages applied to the control terminal of the pMOS transistor 104 would cause voltage drops higher than the supply voltage Vdd, which an LV transistor would not be able to guarantee to sustain.

When it is desired to provide the second voltage of -3 Volts present at the second input terminal INB1 to the output terminal OUT1, the second input terminal INB1 is coupled to the output terminal OUT1, while the first input terminal INA1 (in this case, assumed to be biased to the ground voltage GND) has to be kept electrically insulated therefrom. Thus, the circuital branch on the side of the second input terminal INB1 (in this case, formed by the nMOS transistors 106 and 108) has to be capable of setting up a conductive, low-resistance path from the second input terminal INB1 to the output terminal OUT1, in such a way to provide the voltage of - 3 Volts thereto. For this purpose, the nMOS transistors 106 and 108 have to act as pass transistors with the function of conveying the voltage of -3 Volts. In this case, n-channel MOS transistors may be preferred, because nMOS pass transistors are able to convey negative voltages better than pMOS pass-transistors.

For activating the nMOS transistors 106 and 108, the biasing control block 102 biases the transistors' control terminals to a voltage higher than the voltage (in this case, -3 Volts) that has to be conveyed from the second terminal of the nMOS transistor 108 to the first terminal of the nMOS transistor 106 by an amount of at least the nMOS transistors threshold voltage (in the present example, at least 1 Volt). For example, the biasing control block 102 biases the control terminals of the nMOS transistors 106 and 108 to the ground voltage GND. In this way, when they are activated, the nMOS transistors 106 and 108 have voltage differences across their control terminals and the other terminals thereof that are at most equal to the value of the supply voltage Vdd, so that LV transistors may be used.

As previously stated, when one of the input terminals INA1 and INB1 is coupled to the output terminal OUT1 by the voltage switch 100, the other input terminal has to be kept isolated from the output terminal OUT1.

When the first voltage of 6 Volts is provided to the output terminal OUT1 by activating the pMOS transistor 104, thereby forming a low resistance path on the side of the first input terminal INA1, at least one between the nMOS transistors 106 and 108 has to be turned off. Since in this case the circuital branch on the side of the second input terminal INB1 is connected between a terminal (*i.e.,* the second input terminal INB1) at the ground voltage GND and a terminal (the output terminal OUT1) at 6 Volts, the biasing control block 102 keeps the nMOS transistor 108 *(i.e.,* the transistor connected to the second input terminal INB 1) off, by biasing the nMOS transistor control terminal to the same voltage as the second input terminal INB1, that is, the ground voltage GND. In order be able to use LV transistors only, the biasing control block 102 biases the other nMOS transistors of the circuital branch on the side of the second input terminal INB 1 (in this case, the nMOS transistor 106) to voltage values adapted to avoid voltage differences between their control terminals and their other terminals higher than the supply voltage Vdd. It is irrelevant whether said other nMOS transistors are turned on or off, because the conductive path from the second input terminal INB to the output terminal OUT1 has been already interrupted by ensuring that the transistor 108 is turned off. It has to be appreciated that in the voltage switch 100 the presence of a single nMOS transistor (for example, only the nMOS transistor 108) in the circuital branch on the side of the second input terminal INB1 would not be sufficient, because the voltage difference that occurs between the output terminal OUT1 (in this case, at 6 Volts) and the second input terminal INB1 (in this case, at the ground voltage GND) is higher than the supply voltage Vdd.

When instead the second voltage of -3 Volts is provided to the output terminal OUT1 by activating the nMOS transistors 106 and 108 forming the circuital branch on the side of the second input terminal INB 1, the pMOS transistor 104 has to be turned off. Since in this case the circuital branch on the side of the first input terminal INA1 is connected between a terminal at the ground voltage GND and a terminal at -3 Volts, the pMOS transistor 104 *(i.e.,* the transistor connected to the first input terminal INA1) is kept off by biasing its control terminal to the same voltage as the first input terminal INA1, that is, the ground voltage GND. It has to be appreciated that in this case a single pMOS transistor (the pMOS transistor 104) is sufficient, because the voltage difference between the output terminal OUT1 and the first input terminal is equal, in absolute value, to the supply voltage Vdd.

In greater detail, when it is desired to provide the first voltage of 6 Volts present at the first input terminal INA1 to the output terminal OUT1, the biasing control block 102 biases the control terminals of the pMOS transistor 104 and of the nMOS transistor 106 to a voltage of 3 Volts, and biases the control terminal of the nMOS transistor 108 to the ground voltage GND. In this way, the pMOS transistor 104 is turned on, in such a way that the voltage of the output terminal OUT1 is brought to 6 Volts, as desired. The nMOS transistor 106 has the control terminal at 3 Volts, and the first terminal at 6 Volts. Consequently, the voltage of its second terminal assumes a voltage approximately equal to the control voltage (3 Volts) minus the threshold voltage (1 Volts), *i.e.* 2 Volts. In this way, the nMOS transistor 106 is nearly turned on. The nMOS transistor 108 is instead turned off, because its control terminal is biased to a voltage (0 Volts) that is lower (or, at least equal) with respect those of its first and second terminals. So, the second input terminal INB 1 is electrically insulated from the output terminal OUT1.

When instead it is desired to provide the second voltage of -3 Volts to the output terminal OUT1, the biasing control block 102 biases the control terminals of the pMOS transistor 104 and of the nMOS transistors 106, 108 to the ground voltage GND. In this way, the nMOS transistor 108 is turned on, having the control terminal at a voltage 3 Volts higher with respect to the second terminal. As a consequence, the second terminal of the nMOS transistor 106 is brought to the voltage of-3 Volts too. Since its control terminal is at the ground voltage GND, the nMOS transistor 106 turns on. Thus, the voltage of the output terminal OUT1 is brought to -3 Volts, as desired. The pMOS transistor 104 has the control terminal and the first terminal at the ground voltage GND, and the second terminal at -3 Volts. Consequently, the pMOS transistor 104 is turned off, because its control terminal is biased to a voltage (0 Volts) that is lower with respect those of its first and second terminals. So, the first input terminal INA1 is electrically insulated from the output terminal OUT1.

As can be seen, the voltage values taken by each node of the voltage switch 100 are such as to allow using only LV transistors, without the need of integrating transistors designed to sustain, across at least their control terminal and another terminal, voltage differences higher than the supply voltage Vdd. In fact, in each transistor, the voltage differences across its control terminal and the other terminals are at most equal to 3 Volts *(i.e.,* the value of the supply voltage Vdd).

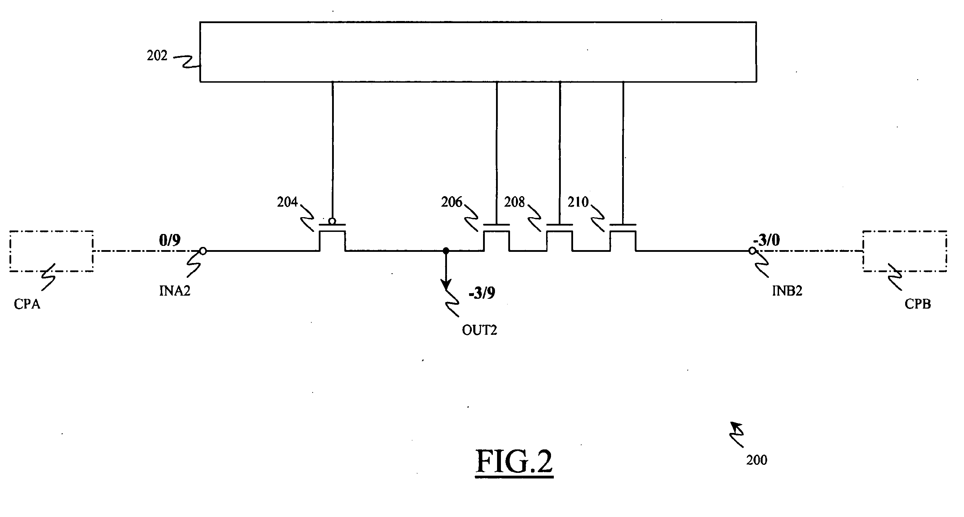

Figure 2 illustrates the electronic circuit schematic according to an embodiment of the invention of a voltage switch 200 having a first input terminal INA2 for receiving a first voltage of, for example, 9 Volts and a second input terminal INB2 for receiving a second voltage of, for example, -3 Volts. The voltage switch 200 further includes an output terminal OUT2 adapted to be selectively coupled with the first input terminal INA2 or with the second input terminal INB2, so as to receive the voltages present thereat, and a biasing control block 202, adapted to bias the MOS transistors included in the voltage switch.

As in the case of the voltage switch 100, the input terminals INA2 and INB2 are assumed to be connected to respective circuital nodes, each of which has a voltage that is assumed to vary between two values. More particularly, when the first input terminal INA2 receives from the respective node the first voltage of 9 Volts, and is coupled to the output terminal OUT2 by the voltage switch 200, the other input terminal INB2 receives from the respective node the ground voltage GND, and is kept insulated from the output terminal OUT2; on the contrary, when the second input terminal INB2 receives from the respective node the second voltage of -3 Volts, and is coupled to the output terminal OUT2 by the voltage switch 200, the other input terminal INA2 receives from the respective node the ground voltage GND, and is kept insulated from the output terminal OUT2.

The voltage switch 200 includes, on the side of the first input terminal INA2, a pMOS transistor 204 having a first terminal connected to the first input terminal INA2, a second terminal connected to the output terminal OUT2 and a control terminal connected to the biasing control block 202. The voltage switch 200 further includes, on the side of the second input terminal INB2 a first nMOS transistor 206 having a first terminal connected to the output terminal OUT2, a second terminal connected to a first terminal of a second nMOS transistor 208 and a control terminal connected to the biasing control block 202. The nMOS transistor 208 has a control terminal connected to the biasing control block 202 and a second terminal connected to a first terminal of a third nMOS transistor 210. The nMOS transistor 210 has a control terminal connected to the biasing control block 202 and a second terminal connected to the second input terminal INB2.

In the same way as for the voltage switch 100, when it is desired to provide the first voltage of 9 Volts present at the first input terminal INA2 to the output terminal OUT2, the first input terminal INA2 is coupled to the output terminal OUT2, while the second input terminal INB2 (in this case, assumed to be biased to the ground voltage GND) has to be kept electrically insulated therefrom. The circuital branch on the side of the first input terminal INA2 is formed by the pMOS transistor 204, having the function of conveying the voltage of 9 Volts from its first terminal to its second terminal. In this case, since said first voltage is equal to 9 Volts, the biasing control block 202 activates the pMOS transistor 204 by biasing the pMOS control terminal to a voltage of 6 Volts.

When it is desired to provide the second voltage of -3 Volts present at the second input terminal INB2 to the output terminal OUT2, the second input terminal INB2 is coupled to the output terminal OUT2, while the first input terminal INA2 (in this case, assumed to be biased to the ground voltage GND) has to be kept electrically insulated therefrom. The circuital branch on the side of the second input terminal INB2 is formed by the nMOS transistors 206, 208 and 210, having the function of providing the voltage of -3 Volts to the output terminal OUT2. Since the second voltage is equal to -3 Volts, as in the voltage switch 100, the biasing control block 202 activates the nMOS transistors 206, 208 and 210 biasing the nMOS transistor control terminals to the ground voltage GND.

When the first voltage of 9 Volts is provided to the output terminal OUT2 by activating the pMOS transistor 204, at least one between the nMOS transistors 206, 208 and 210 has to be turned off. For this purpose, the biasing control block 202 keeps the nMOS transistor 210 off by biasing the nMOS transistor control terminal to the same voltage as the second input terminal INB2, that is, the ground voltage GND. As in the case of the voltage switch 100, it is irrelevant if the other nMOS transistors 206, 208 of the circuital branch are turned on or off, but the biasing control block 202 biases them to voltage values such to avoid voltage differences between their control terminals and their other terminals higher than the supply voltage Vdd, so as to allow the use of LV transistors. It has to be appreciated that in this case the voltage difference between the output terminal OUT2 and the second input terminal INB2 is equal to 9 Volts. Consequently, the circuital branch on the side of the second input terminal INB2 has to include at least three LV nMOS transistors 206, 208, 210, each one capable of sustaining a voltage difference of 3 Volts.

When instead the second voltage of -3 Volts is provided to the output terminal OUT2 by activating the nMOS transistors 206, 208, 210, the pMOS transistor 204 is kept off by biasing its control terminal to the same voltage as the first input terminal INA2, that is, the ground voltage GND. In this case, the circuital branch on the side of the first input terminal INA2 includes only a single pMOS transistor 204, because the voltage difference between the output terminal OUT2 and the first input terminal INA2 is equal in absolute value to the supply voltage Vdd.

In greater detail, when it is desired to provide the voltage of 9 Volts to the output terminal OUT2, the biasing control block 202 biases the control terminals of the pMOS transistor 204 and of the nMOS transistor 206 to a voltage of 6 Volts, the control terminal of the nMOS transistor 208 to a voltage of 3 Volts and the control terminal of the nMOS transistor 210 to the ground voltage GND. In this way, the pMOS transistor 204 is turned on, establishing a conductive path between the first input terminal INA2 and the output terminal OUT2, in such a way that the voltage of the output terminal OUT2 is brought to 9 Volts, as desired. The nMOS transistor 206 has the control terminal at 6 Volts, and the first terminal at 9 Volts. Consequently, the voltage of its second terminal assumes a voltage approximately equal to the control voltage (6 Volts) minus the threshold voltage (1 Volt), *i.e.* 5 Volts. In this way, the nMOS transistor 206 is nearly turned on. Similarly, since the nMOS transistor 208 has the control terminal at 3 Volts and the first terminal at 5 Volts, the voltage of its second terminal assumes a voltage approximately equal to 2 Volts, *i.e.,* equal to the control voltage (3 Volts) minus the threshold voltage. The nMOS transistor 210 is instead turned off, because its control terminal is biased to a voltage (0 Volts) that is lower (or, at most, equal) with respect the voltage of its first and second terminals. So, the second input terminal INB2 is electrically insulated from the output terminal OUT2.

When it is desired to provide the voltage of -3 Volts to the output terminal OUT2, the biasing control block 102 biases the control terminals of the pMOS transistor 204 and of the nMOS transistors 206, 208 and 210 to the ground voltage GND. In this way, the nMOS transistor 210 is turned on, having the control terminal at a voltage 3 Volts higher with respect to the second terminal. As a consequence, the second terminal of the nMOS transistor 208 is brought to the voltage of -3 Volts too. In the same way, both the nMOS transistor 208 and 206 are turned on. Thus, a conductive path is established between the second input terminal INB2 and the output terminal OUT2, in such a way that the voltage of the output terminal OUT2 is brought to -3 Volts, as desired. The pMOS transistor 204 has the control terminal and the first terminal at the ground voltage GND, and the second terminal at -3 Volts. Consequently, the pMOS transistor 204 is turned off. So, the first input terminal INA2 is electrically insulated from the output terminal OUT2.

As can be seen, the voltage values taken by each node of the voltage switch 200 are such as to allow using only LV transistors, without the need of integrating transistors designed to sustain, across at least their control terminal and another of their terminals, voltage differences higher than the supply voltage Vdd. In fact, in each transistor, the voltage differences across its control terminal and the other terminals are at most equal to 3 Volts.

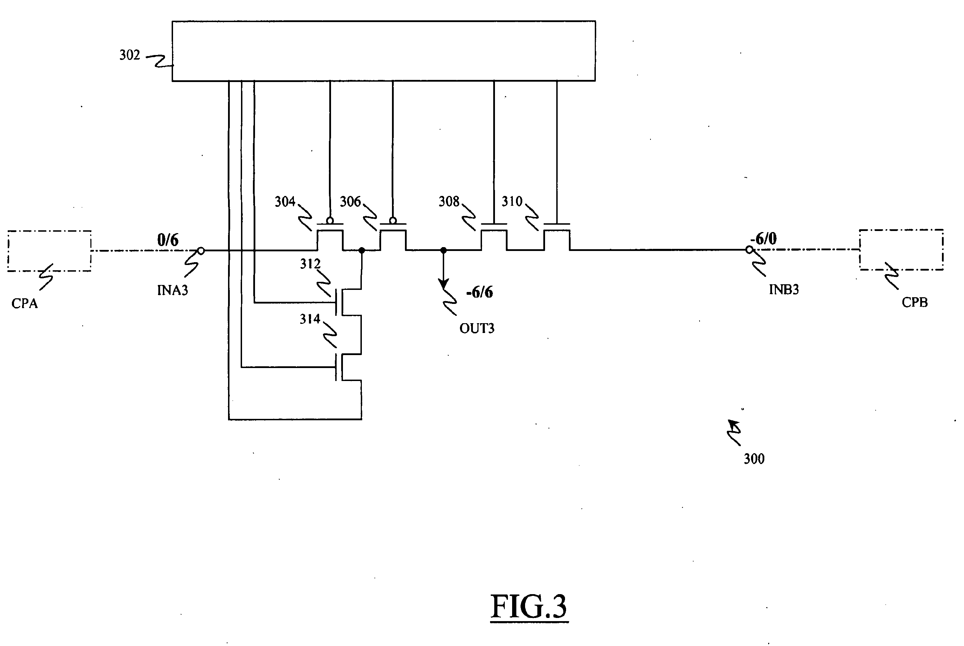

Figure 3 illustrates the electronic circuit schematic according to an embodiment of the invention of a voltage switch 300 having a first input terminal INA3 for receiving a first voltage of, for example, 6 Volts and a second input terminal INB3 for receiving a second voltage of, for example, -6 Volts. The voltage switch 300 further includes an output terminal OUT3 adapted to be selectively coupled with the first input terminal INA3 or with the second input terminal INB3, so as to receive the voltages present thereat, and a biasing control block 302, adapted to bias the MOS transistors included in the voltage switch. The input terminals INA3 and INB3 are connected to respective circuital nodes. When the first input terminal INA3 receives from the respective node the first voltage of 6 Volts, the other input terminal INB3 receives from the respective node the ground voltage GND, and is kept insulated from the output terminal OUT3; on the contrary, when the second input terminal INB3 receives from the respective node the second voltage of -6 Volts, the other input terminal INA3 receives from the respective node the ground voltage GND, and is kept insulated from the output terminal OUT3.

The voltage switch 300 includes, on the side of the first input terminal INA3, a first pMOS transistor 304 having a first terminal connected to the first input terminal INA3, a second terminal connected to a first terminal of a second pMOS transistor 306 and a control terminal connected to the biasing control block 302. The pMOS transistor 306 has a control terminal connected to the biasing control block 302 and a second terminal connected to the output terminal OUT3. The voltage switch 300 further includes on the side of the second input terminal INB3, a first nMOS transistor 308 having a first terminal connected to the output terminal OUT3, a second terminal connected to a first terminal of a second nMOS transistor 310 and a control terminal connected to the biasing control block 302. The nMOS transistor 310 has a control terminal connected to the biasing control block 302 and a second terminal connected to the second input terminal INB3. The voltage switch 300 further includes a third nMOS transistor 312 having a first terminal connected to the first terminal of the pMOS transistor 306, a control terminal connected to the biasing control block 302 and a second terminal connected to a first terminal of a fourth nMOS transistor 314. The nMOS transistor 314 has a control terminal and a second terminal that are connected to the biasing control block 302.

The circuital branch on the side of the first input terminal INA3 is formed by the pMOS transistors 304, 306, having the function of conveying the first voltage of 6 Volts from the first input terminal INA3 to the output terminal OUT3. In this case, since said first voltage is equal to 6 Volts, the biasing control block 302 activates the pMOS transistors 304 and 306 by biasing pMOS control terminals to a voltage of 3 Volts.

The circuital branch on the side of the second input terminal INB3 is formed by the nMOS transistors 308, 310, having the function of providing the second voltage of -6 Volts to the output terminal OUT3. In this case, since the second voltage is equal to -6 Volts, the biasing control block 302 activates the nMOS transistors 308 and 310 by biasing the nMOS transistor control terminals to a voltage of-3 Volts.

When the first voltage of 6 Volts is provided to the output terminal OUT3 by activating the pMOS transistors 304 and 306, at least one between the nMOS transistors 308 and 310 has to be turned off. For this purpose, the biasing control block 302 keeps the nMOS transistor 310 connected to the second input terminal INB3 off by biasing the nMOS transistor control terminal to the same voltage of the second input terminal INB3, that is, the ground voltage GND. As in the previous cases, it is irrelevant whether the other nMOS transistor 308 of the circuital branch is turned on or off, but the biasing control block 302 biases the nMOS transistor 308 to voltage value such as to avoid voltage differences between its control terminal and the other terminals higher than the supply voltage Vdd, so as to allow the use of an LV transistor. It has to be appreciated that in this case the voltage difference that occurs between the output terminal OUT3 and the second input terminal is equal to 6 Volts. Consequently, the circuital branch on the side of the second input terminal INB3 has to include at least two LV nMOS transistors 308 and 310, each one capable of sustaining a voltage difference of 3 Volts.

When instead the second voltage of -6 Volts is provided to the output terminal OUT3 by activating the nMOS transistors 308 and 310, the biasing control block 302 keeps the pMOS transistor 304 off by biasing the pMOS control terminal to the same voltage as the first input terminal INA3, that is, the ground voltage GND. Again, the biasing control block 302 biases the remaining pMOS transistor 306 of the branch to a voltage value such as to avoid voltage differences between its control terminal and the other terminals higher than the supply voltage Vdd. In this case, the circuital branch on the side of the first input terminal INA3 has to include at least two pMOS transistor 304 and 306, because the voltage difference between the output terminal OUT3 and the first input terminal INA3 is equal in absolute value to twice the supply voltage Vdd.

In greater detail, when it is desired to provide the voltage of 6 Volts to the output terminal OUT3, the biasing control block 302 biases the control terminals of the pMOS transistors 304, 306 and of the nMOS transistors 308, 312 to a voltage of 3 Volts, and biases the control terminals of the nMOS transistors 310, 314 to the ground voltage GND. In this way, the pMOS transistor 304 is turned on, so as to provide a voltage of 6 Volts to the first terminal of the pMOS transistor 306. Since its control terminal is at 3 Volts, also the pMOS transistor 306 is turned on, establishing a conductive path between the first input terminal INA3 and the output terminal OUT3, in such a way that the voltage of the output terminal OUT3 is brought to 6 Volts, as desired. The circuital branch formed by the nMOS transistors 312 and 314 is instead deactivated. In fact, the nMOS transistor 314 is turned off, while the nMOS transistor 312 is nearly turned off (its second terminal assumes a voltage of about 2 Volts, i.e., equal to the control terminal voltage minus the threshold voltage). Moreover, the nMOS transistor 310 is turned off, and the nMOS transistor 308 is nearly turned on (its second terminal being at approximately 2 Volts).

When it is desired to provide the voltage of -6 Volts to the output terminal OUT3, the biasing control block 302 biases the control terminal of the pMOS transistor 304 and of the nMOS transistors 312, 314 to the ground voltage GND, and the control terminals of the pMOS transistor 306 and of the nMOS transistors 308, 310 to a voltage of -3 Volts. Moreover, the biasing control block 302 biases the second terminal of the nMOS transistor 314 to a voltage of -3 Volts. In this way, the nMOS transistor 310 is turned on, having the control terminal at a voltage that is 3 Volts higher with respect to the second terminal. As a consequence, the second terminal of the nMOS transistor 308 is brought to the voltage of -6 Volts. Since its control terminal is at -3 Volts, the nMOS transistor 308 turns on. Thus, a conductive path is established between the second input terminal INB3 and the output terminal OUT3, in such a way that the voltage of the output terminal OUT3 is brought to -6 Volts, as desired. The nMOS transistor 314 is turned on, because its control terminal is at a voltage that is 3 Volts higher with respect to the second terminal. As a consequence, the first terminal of the nMOS transistor 314 is brought to -3Volts, the nMOS transistor 312 is turned on and the first terminal of the pMOS transistor 306 is brought to -3 Volts, too. Since its first terminal and its control terminal are at the same voltage (-3 Volts), the pMOS transistor 306 is turned off, acting as a reverse-biased diode. Similarly, also the pMOS 304 is turned off, and the first input terminal INA3 is electrically insulated from the output terminal OUT3.

Even in this case, the voltage values taken by each node of the voltage switch 300 are such as to allow using only LV transistors. In fact, in each transistor, the voltage differences across its control terminal and the other terminals are at most equal to 3 Volts.

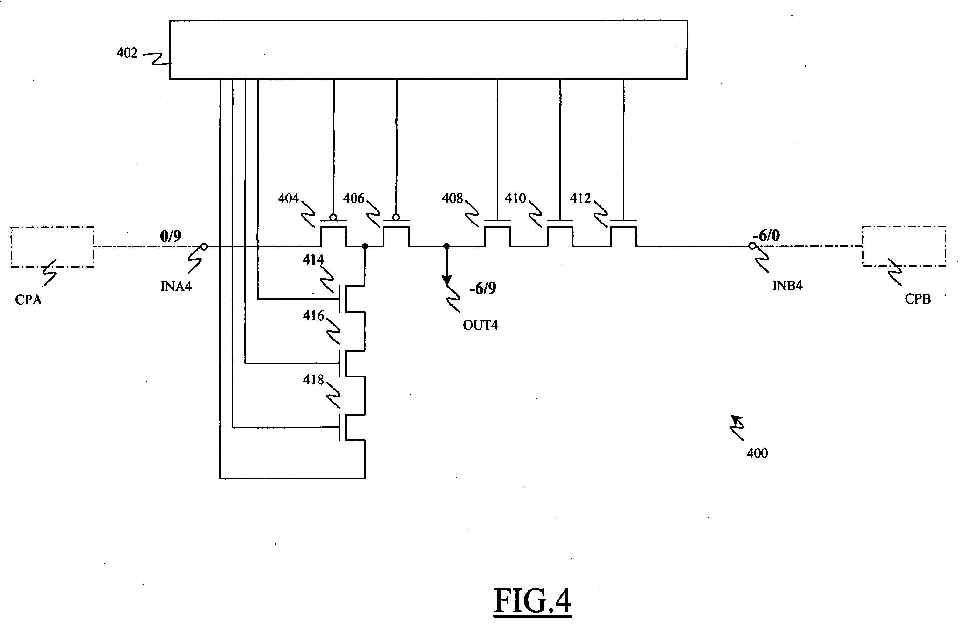

Figure 4 illustrates the electronic circuit schematic according to an embodiment of the invention of a voltage switch 400 having a first input terminal INA4 for receiving a first voltage of, for example, 9 Volts and a second input terminal INB4 for receiving a second voltage of, for example, -6 Volts. The voltage switch 400 further includes an output terminal OUT4 adapted to be selectively coupled with the first input terminal INA4 or with the second input terminal INB4, so as to receive the voltages present thereat, and a biasing control block 402, adapted to bias the MOS transistors included in the voltage switch. The input terminals INA4 and INB4 are connected to respective circuital nodes. When the first input terminal INA4 receives from the respective node the first voltage of 9 Volts, the other input terminal receives from the respective node the ground voltage GND, and is kept insulated from the output terminal OUT4; on the contrary, when the second input terminal INB4 receives from the respective node the second voltage of -6 Volts, the other input terminal receives from the respective node the ground voltage GND, and is kept insulated from the output terminal OUT4.

The voltage switch 400 includes, on the side of the first input terminal INA4, a first pMOS transistor 404 having a first terminal connected to the first input terminal INA4, a second terminal connected to a first terminal of a second pMOS transistor 406 and a control terminal connected to the biasing control block 402. The pMOS transistor 406 has a control terminal connected to the biasing control block 402 and a second terminal connected to the output terminal OUT4. The voltage switch 400 further includes, on the side of the first input terminal INA4, a first nMOS transistor 408 having a first terminal connected to the output terminal OUT4, a second terminal connected to a first terminal of a second nMOS transistor 410 and a control terminal connected to the biasing control block 402. The nMOS transistor 410 has a control terminal connected to the biasing control block 402 and a second terminal connected to a first terminal of a third nMOS transistor 412. The nMOS transistor 412 has the control terminal connected to the biasing control block 402 and the second terminal connected to the second input terminal INB4. The voltage switch 400 further includes a fourth nMOS transistor 414 having a first terminal connected to the first terminal of the pMOS transistor 406, a control terminal connected to the biasing control block 402 and a second terminal connected to a first terminal of a fifth nMOS transistor 416. The nMOS transistor 416 has a control terminal connected to the biasing control block 402 and a second terminal that is connected to a first terminal of a sixth nMOS transistor 418. The nMOS transistor 418 has the control and the second terminals that are connected to the biasing control block 402.

The circuital branch on the side of the first input terminal INA4 is formed by the pMOS transistors 404, 406 having the function of conveying the first voltage of 9 Volts from the first input terminal INA4 to the output terminal OUT4. In this case, since said first voltage is equal to 9 Volts, the biasing control block 402 activates the pMOS transistors 404 and 406 by biasing the pMOS transistors control terminals to a voltage of 6 Volts.

The circuital branch on the side of the second input terminal INB4 is formed by the nMOS transistors 408, 410 and 412, having the function of providing the second voltage of -6 Volts to the output terminal OUT4. In this case, since the second voltage is equal to -6 Volts, the biasing control block 402 activates the nMOS transistors 408, 410 and 412 by biasing the nMOS transistors control terminals to a voltage of-3 Volts.

When the first voltage of 9 Volts is provided to the output terminal OUT4 by activating the pMOS transistors 404 and 406, at least one among the nMOS transistors 408, 410 and 412 has to be turned off. For this purpose, the biasing control block 402 keeps the nMOS transistor 412 off by biasing the nMOS control terminal to the same voltage of the second input terminal, that is, the ground voltage GND. The biasing control block 402 biases the remaining nMOS transistors 410 and 408 of the circuital branch to voltage values such as to avoid voltage differences between their control terminals and their other terminals higher than the supply voltage Vdd. It can be appreciated that in this case the voltage difference between the output terminal OUT4 and the second input terminal is equal to 9 Volts. Consequently, the circuital branch on the side of the second input terminal INB4 has to include at least three LV nMOS transistors 408, 410 and 412, each one capable of sustaining a voltage difference of 3 Volts.

When instead the second voltage of -6 Volts is provided to the output terminal OUT4 by activating the nMOS transistors 408, 410 and 412, the biasing control block 402 keeps the pMOS transistor 404 off by biasing the pMOS control terminal to the same voltage of the first input terminal, that is, the ground voltage GND. Again, the biasing control block 402 biases the control terminal of the remaining pMOS transistor 406 of the branch to a voltage value such as to avoid voltage differences between its control terminal and the other terminals higher than the supply voltage Vdd. In this case, the circuital branch on the side of the first input terminal INA4 has to include at least two pMOS transistor 404 and 406, because the voltage difference between the output terminal OUT4 and the first input terminal INA4 is equal in absolute value to twice the supply voltage Vdd.

In greater detail, when it is desired to provide the first voltage of 9 Volts to the output terminal OUT4, the biasing control block 402 biases the control terminals of the pMOS transistors 404, 406 and of the nMOS transistors 408, 414 to a voltage of 6 Volts, the control terminals of the nMOS transistors 410, 416 to a voltage of 3Volts, and the control terminals of the nMOS transistors 412, 418 to the ground voltage GND. Moreover, the biasing control block 402 biases the second terminal of the nMOS transistor 418 to the ground voltage GND too. In this way, the pMOS transistor 404 is turned on, so as to provide a voltage of 9 Volts to the first terminal of the pMOS transistor 406. Since its control terminal is at 6 Volts, also the pMOS transistor 406 is turned on, establishing a conductive path between the first input terminal INA4 and the output terminal OUT4, in such a way that the voltage of the output terminal OUT4 is brought to 9 Volts, as desired. The circuital branch formed by the nMOS transistors 414, 416 and 418 is instead deactivated. In fact, the nMOS transistor 418 is turned off, while the nMOS transistors 416 and 418 are nearly turned off (their second terminal assume a voltage of about the control terminal voltage minus the threshold voltage, *i.e.,* 2 Volts and 5 Volts, respectively). Moreover, the nMOS transistor 412 is turned off, and the nMOS transistors 408 and 410 are nearly turned off (their second terminals being at approximately 5 and 2 Volts, respectively).

When it is desired to provide the second voltage of -6 Volts to the output terminal OUT4, the biasing control block 402 biases the control terminals of the pMOS transistor 406 and of the nMOS transistors 408, 410, 412 to a voltage of -3 Volts and the control terminals of the pMOS transistor 404 and of the nMOS transistors 414, 416, 418 to the ground voltage GND. Moreover, the biasing control block 402 biases the second terminal of the nMOS transistor 418 to the ground voltage GND. In this way, the nMOS transistor 412 is turned on, having the control terminal at a voltage that is 3 Volts higher with respect to the second terminal. As a consequence, the second terminal of the nMOS transistor 410 is brought to the voltage of-6 Volts. Since its control terminal is at -3 Volts, the nMOS transistor 410 turns on, providing a voltage of -6 Volts to the second terminal of the nMOS transistor 408. In the same way, the nMOS transistor 408 turns on. Thus, a conductive path is established between the second input terminal INB4 and the output terminal OUT4, in such a way that the voltage of the output terminal OUT4 is brought to -6 Volts, as desired. The nMOS transistor 418 is turned on, because its control terminal is at a voltage that is 3 Volts higher with respect to the second terminal. As a consequence, the first terminal of the nMOS transistor 418 is brought to -3Volts, the nMOS transistor 416 is turned on and the second terminal of the nMOS transistor is brought to -3 Volts. The latter voltage value allows the nMOS 414 to be turned on, biasing the second terminal of the pMOS transistor 404 to -3 Volts. Since its first terminal and its control terminal are at the same voltage (-3 Volts), the pMOS transistor 406 is turned off, acting as a reverse-biased diode. Similarly, the pMOS transistor 404 is turned off, and the first input terminal INA4 is electrically insulated from the output terminal OUT4.

Even in this case, the voltage values taken by each node of the voltage switch 400 are such as to allow using only LV transistors. In fact, in each transistor, the voltage differences across its control terminal and the other terminals are at most equal to the supply voltage Vdd.

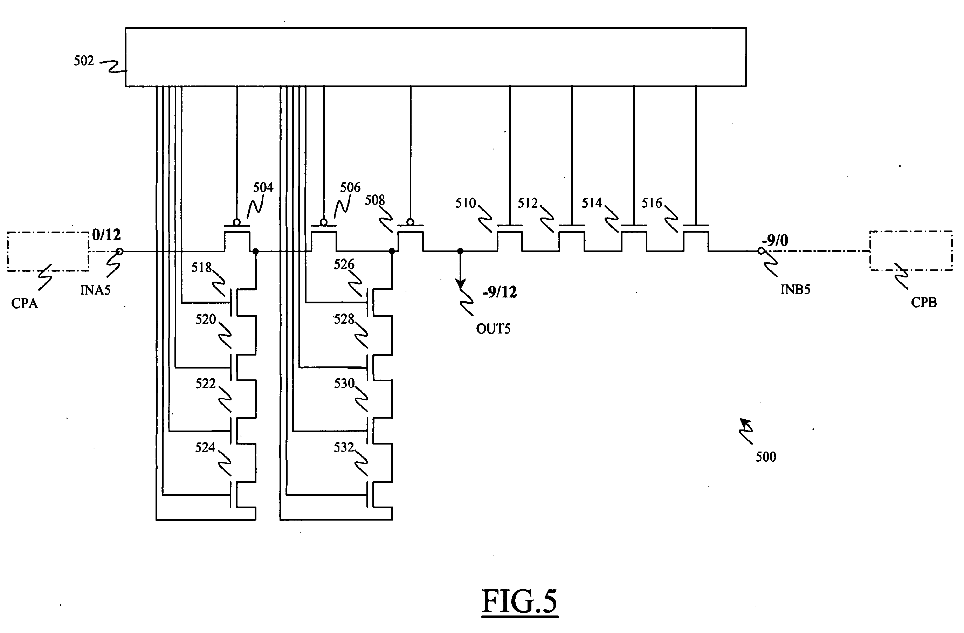

Figure 5 illustrates the electronic circuit schematic according to an embodiment of the invention of a voltage switch 500, having a first input terminal INA5 for receiving a first voltage of, for example, 12 Volts and a second input terminal INB5 for receiving a second voltage of, for example, -9 Volts. The voltage switch 500 further includes an output terminal OUT5 adapted to be selectively coupled with the first input terminal INA5 or with the second input terminal INB5, so as to receive the voltages present thereat, and a biasing control block 502, adapted to bias the MOS transistors included in the voltage switch. The input terminals INA5 and INB5 are connected to respective circuital nodes. When the first input terminal INA5 receives from the respective node the first voltage of 12 Volts, the other input terminal receives from the respective node the ground voltage GND, and is kept insulated from the output terminal OUT5; differently, when the second input terminal INB5 receives from the respective node the second voltage of -9 Volts, the other input terminal receives from the respective node the ground voltage GND, and is kept insulated from the output terminal OUT5.

The voltage switch 500 includes, on the side of the first input terminal INA5, a first pMOS transistor 504 having a first terminal connected to the first input terminal INA5, a second terminal connected to a first terminal of a second pMOS transistor 506 and a control terminal connected to the biasing control block 502. The pMOS transistor 506 has a control terminal connected to the biasing control block 502 and a second terminal connected to a first terminal of a third pMOS transistor 508. The pMOS transistor 508 has a control terminal connected to the biasing control block 502 and a second terminal connected to the output terminal OUT5. The voltage switch 500 further includes, on the side of the second input terminal INB5, a first nMOS transistor 510 having a first terminal connected to the output terminal OUT5, a second terminal connected to a first terminal of a second nMOS transistor 512 and a control terminal connected to the biasing control block 502. The nMOS transistor 512 has a control terminal connected to the biasing control block 502 and a second terminal connected to a first terminal of a third nMOS transistor 514, the latter transistor having a control terminal connected to the biasing control block 502 and a second terminal connected to a first terminal of a fourth nMOS transistor 516. The nMOS transistor 516 has a control terminal connected to the biasing control block 502 and a second terminal connected to the second input terminal INB5. The voltage switch 500 further includes a first and a second circuital branches, connected to the second terminal of the pMOS transistor 504 and to the second terminal of the pMOS transistor 506, respectively. The first circuital branch is formed by four nMOS transistors 518, 520, 522, 524 connected in series, with a first nMOS transistor 518 in the series having a first terminal connected to the second terminal of the pMOS transistor 504 and an nMOS transistor 524 in the series having a second terminal connected to the biasing control block 502. The nMOS transistors 518, 520, 522, 524 have the control terminals connected to the biasing control block 502. In the same way, the second circuital branch is formed by four nMOS transistors 526, 528, 530, 532 connected in series, with a first nMOS transistor 526 in the series having a first terminal connected to the second terminal of the pMOS transistor 506 and a last nMOS transistor 532 in the series having a second terminal connected to the biasing control block 502. The nMOS transistors 526, 528, 530, 532 have the control terminals connected to the biasing control block 502.

The circuital branch on the side of the first input terminal INA5 is formed by the pMOS transistors 504, 506 and 508, having the function of conveying the first voltage of 12 Volts from the first input terminal INA5 to the output terminal OUT5. In this case, since said first voltage is equal to 12 Volts, the biasing control block 502 activates the pMOS transistors 504, 506 and 508 by biasing the pMOS transistors control terminals to a voltage of 9 Volts.

The circuital branch on the side of the second input terminal INB5 is formed by the nMOS transistors 510, 512, 514 and 516, having the function of providing the second voltage of -9 Volts to the output terminal OUT5. In this case, since the second voltage is equal to -9 Volts, the biasing control block 502 activates the nMOS transistors 510, 512, 514 and 516 by biasing the nMOS transistors control terminals to a voltage of-6 Volts.

When the first voltage of 12 Volts is provided to the output terminal OUT5 by activating the pMOS transistors 504, 506 and 508, at least one between the nMOS transistors 510, 512, 514 and 516 has to be turned off. For this purpose, the biasing control block 502 keeps the nMOS transistor 516 off by biasing the nMOS transistor control terminal to the same voltage as the second input terminal, that is, the ground voltage GND. The biasing control block 502 biases the remaining nMOS transistors 510, 512 and 514 of the circuital branch to voltage values such to avoid voltage differences between their control terminals and their other terminals higher than the supply voltage Vdd. It has to be appreciated that in this case the voltage difference between the output terminal OUT5 and the second input terminal INB5 is equal to 12 Volts. Consequently, the circuital branch on the side of the second input terminal INB5 has to include at least four LV nMOS transistors 510, 512, 514 and 516, each one capable of sustaining a voltage difference of 3 Volts.

When instead the second voltage of -9 Volts is provided to the output terminal OUT5 by activating the nMOS transistors 510, 512, 514 and 516, the biasing control block 502 keeps the pMOS transistor 504 off by biasing the pMOS control terminal to the same voltage as the first input terminal INA5, that is, the ground voltage GND. Again, the biasing control block 502 biases the remaining pMOS transistors 506 and 508 of the branch to a voltage value such to avoid voltage differences between their control terminal and the other terminals higher than the supply voltage Vdd. In this case, the circuital branch on the side of the first input terminal INA5 has to include at least three pMOS transistor 504, 406 and 508, because the voltage difference between the output terminal OUT5 and the first input terminal INA5 is equal in absolute value to three times the supply voltage Vdd.

In greater detail, when it is desired to provide the first voltage of 12 Volts to the output terminal OUT5, the biasing control block 502 biases the control terminals of the pMOS transistors 504, 506, 508 and of the nMOS transistors 510, 518, 526 to a voltage of 9 Volts, the control terminals of the nMOS transistors 512, 520, 528 to a voltage of 6Volts, the control terminals of the nMOS transistors 5514, 522, 530 to a voltage of 3 Volts and the nMOS transistors 516, 524, 532 to the ground voltage GND. Moreover, the biasing control block 402 biases the second terminals of the nMOS transistors 524, 532 to the ground voltage GND too. In this way, the pMOS transistors 504, 506 and 508 are turned on, so as to establish a conductive path between the first input terminal INA5 and the output terminal OUT5, in such a way that the voltage of the output terminal OUT5 is brought to 12 Volts, as desired. The first circuital branch formed by the nMOS transistors 518, 520, 522, 524 is instead deactivated. In fact, the nMOS transistor 524 is turned off, while the nMOS transistors 522, 520, 518 are nearly turned off (their second terminals assume a voltage of about the control terminal voltage minus the threshold voltage, *i.e.,* 2, 5 and 8 Volts, respectively). The nMOS transistors 526, 528, 530, 532 of the second circuital branch act in the same way, with the same biasing voltages. Moreover, the nMOS transistor 516 is turned off, and the nMOS transistors 510, 512, 514 are nearly turned off (their second terminals being at approximately 8, 5 and 2 Volts, respectively).

When it is desired to provide the second voltage of -9 Volts to the output terminal OUT5, the biasing control block 502 biases the control terminal of the pMOS transistor 508 and of the nMOS transistors 510, 512, 514, 516 to a voltage of -6 Volts, the control terminals of the pMOS transistor 506 and of the nMOS transistors 526, 528, 530, 532 to a voltage of -3 Volts and the control terminals of the pMOS transistor 504 and of the nMOS transistors 518, 520, 522, 524 to the ground voltage GND. Moreover, the biasing control block 502 biases the second terminal of the nMOS transistor 524 to a voltage of -3 Volts and the second terminal of the nMOS transistor 532 to a voltage of -6 Volts. In this way, the nMOS transistors 510, 512, 514, 516 are turned on, so as to establish a conductive path between the second input terminal INB5 and the output terminal OUT5, in such a way that the voltage of the output terminal OUT5 is brought to -9 Volts, as desired. Having the control terminals biased to a voltage of -3 Volts, the nMOS transistors 526, 528, 530, 532 are all turned on. As a consequence, the second terminal of the pMOS transistor 506 is biased to -6 Volts. Since its first terminal and its control terminal are at the same voltage (-6 Volts), the pMOS transistor 508 is turned off, acting as a reverse-biased diode. In the same way, the nMOS transistors 518, 520, 522, 524 are all turned on, because they have the control terminals biased to the ground voltage GND. Thus, the second terminal of the pMOS transistor 504 is biased to -3 Volts. Even in this case, since its first terminal and its control terminal are at the same voltage (-3 Volts), the pMOS transistor 506 is turned off, acting as a reverse-biased diode. Moreover, the pMOS transistor 504 is turned off, and the first input terminal INA5 is electrically insulated from the output terminal OUT5.

Also in this case, the voltage values taken by each node of the voltage switch 500 are such as to allow using only LV transistors. In fact, in each transistor, the voltage differences across its control terminal and the other terminals are at most equal to the supply voltage Vdd.

It can be appreciated that most of the biasing voltages provided by the biasing control blocks of the voltage switches previously described are higher than the supply voltage Vdd. Consequently, the biasing control blocks may have to include boosting circuits adapted to generate such high voltages. In alternative, if the IC wherein the voltage switch(es) is(are) included includes one or more charge pumps, the high voltages necessary to the biasing control block(s) may be provided by the charge pump(s).

According to an embodiment of the present invention, the biasing control block of a voltage switch may expediently include other voltage switches, used for providing the high voltages thereto. For example, referring to the voltage switch 500, it can be observed that the biasing control terminal 502 biases the control terminal of the pMOS transistor 508 to 9 Volts when the first input terminal INA5 is coupled to the output terminal OUT5, and to -6 Volts when the first input terminal INA5 is kept insulated from the output terminal OUT5. Consequently, since the output terminal OUT4 of the voltage switch 400 is capable of assuming both 9 and -6 Volts, the latter voltage switch 400 may be included into the biasing control block 502, with the output terminal OUT4 connected to the control terminal of the pMOS transistor 508.

It has to be considered that, when the voltage switches previously described switch (*i.e.*, pass from a condition in which the first input terminal is coupled with the output terminal to a condition wherein the second input terminal is coupled with the output terminal, and vice versa), the biasing control block should switch the biasing voltages with a delay as low as possible. In fact, if the voltage provided to a control terminal of an LV transistor included in a voltage switch is switched (even slightly) later compared to the voltages provided to the control terminals of the other LV transistors, a dangerous voltage difference higher than the supply voltage Vdd between terminals of the LV transistors may be incurred. If the high voltages necessary to the biasing control blocks are obtained from a charge pump, it is more likely that the bias voltages generated are capable to switch synchronously.

Having described in details several exemplary voltage switches, with reference to Figure 6 a more general guideline will be now described for designing a generic two-inputs voltage switch 600 capable of receiving a generic pair of voltage values at its inputs that make use of LV transistors only.

As in the previous cases, the voltage switch 600 includes a first and a second input terminals INA, INB and an output terminal OUT adapted to be selectively coupled with the first input terminal INA or with the second input terminal INB. The first input terminal INA is assumed to be connected to a circuital node whose voltage varies between a first input voltage nVdd and, for example, the ground voltage GND. The first input voltage nVdd is a multiple of the supply voltage Vdd; more particularly, the first input voltage nVdd is equal to the supply voltage Vdd multiplied by a first (positive or negative) integer coefficient n. The second input terminal INB is assumed to be connected to a circuital node whose voltage varies between a second input voltage mVdd and, for example, the ground voltage GND. The second input voltage mVdd is a multiple of the supply voltage Vdd; more particularly, the second input voltage mVdd is equal to the supply voltage Vdd multiplied by a second (positive or negative) integer coefficient m. When the first input terminal INA is biased to the first input voltage nVdd, the second input terminal INB is biased to the ground voltage GND, while, when the second input terminal INB is biased to the second input voltage mVdd, the first input terminal INA is biased to the ground voltage GND. For the sake of simplicity, it is assumed that the first integer n is greater than the second integer m.

The voltage switch 600 includes a first circuital branch 610 formed by a number of pMOS transistors P(i) (i=1 to m) connected in series equal to the absolute value of the second integer m. Said first circuital branch 610 connect the first input terminal INA with the output terminal OUT. In the same way, a second circuital branch 620 connects the second input terminal INB with the output terminal OUT. The second circuital branch 620 includes a number of nMOS transistors N(j) (j=1 to n) connected in series equal to the absolute value of the first integer n. It is pointed out that all the voltage switches previously described fall in the category represented by this generic voltage switch 600. For example, the voltage switch 100 described in Figure 1 is equal to that of Figure 6 if the first integer n is equal to 2 (in fact, its first input terminal INA1 is adapted to receive 2Vdd = 6 Volts) and the second integer m is equal to -1 (its second input terminal INB1 is adapted to receive -Vdd = -3 Volts).

If the output terminal OUT has to be coupled with the first input terminal INA for receiving the first input voltage nVdd, all the m pMOS transistors P(i) of the first circuital branch 610 have to be turned on. In this way, each pMOS acts as a pass-transistor capable of providing the voltage received at its first terminal to its second terminal (in Figure 6, the first terminal of each transistor (both pMOS and nMOS) is that on the left of the transistor itself, while the second terminal is that on the right thereof), and a conductive path from the first input terminal INA to the output terminal OUT is established. In order for the pMOS transistors P(i) of the first circuital branch 610 to be turned on, so as to provide the first input voltage nVdd to the output terminal OUT, the control terminals of all the pMOS transistors P(i) may be expediently biased to a voltage equal to (n-1)Vdd. In this way, the voltage differences across the control terminal of a generic pMOS P(i) transistor and the other terminals thereof are at most equal the value of the supply voltage Vdd. Thanks to this, the first circuital branch 610 can be realized using only LV transistors. Conversely, the nMOS transistors N(j) of the second circuital branch 620 have to be biased in such a way to electrically insulate the second input terminal INB from the output terminal OUT. Since the second input terminal INB is biased to the ground voltage GND, the second circuital branch 620 is subjected to a voltage difference (from the terminal connected to the output terminal OUT to the second input terminal INB) equal to the first input voltage nVdd. However, in order to use LV transistors only, each nMOS transistor N(j) of the second circuital branch 620 shall experience voltage differences across its control terminal and the other terminals thereof that are at most equal the value of the supply voltage Vdd. Thus, each nMOS transistor N(j) is biased in such a way that the total voltage drop between the output terminal OUT and the second input terminal INB (equal to the first input voltage nVdd) is equally distributed across all the nMOS transistors. Since the number of nMOS transistors N(j) included into the second circuital branch 620 is equal to the (absolute value of the) first integer n, by biasing the transistors' control terminals to voltages starting from (n-1)Vdd for the nMOS transistor N(1) (connected to the output terminal OUT), and proceeding to voltages lower and lower for the following nMOS transistors in the branch (for example, by reducing the value of Vdd per each transistor), it is possible to ensure that each nMOS transistor N(j) is subjected to a voltage difference at most equal to the supply voltage Vdd, as desired. With this biasing scheme, the nMOS transistor N(n) (connected to the second input terminal INB) is surely turned off, having the control terminal at a voltage equal to that of the second input terminal INB *(i.e.,* the ground voltage GND).

If the output terminal OUT has to be coupled with the second input terminal INB for receiving the second input voltage mVdd, all the m nMOS transistors N(j) of the second circuital branch 620 have to be turned on. In this way, each nMOS transistor N(j) acts as a pass-transistors capable of providing the voltage received at its second terminal to its first terminal, and a conductive path from the second input terminal INB to the output terminal OUT is established. In order for the nMOS transistors N(j) of the second circuital branch 620 to be turned on, so as to provide the second input voltage mVdd to the output terminal OUT, the control terminals of all the nMOS transistors N(j) may be expediently biased to a voltage equal to (m+1)Vdd. In this way, the voltage differences across the control terminal of a generic nMOS transistor N(j) and the other terminals thereof are at most equal the value of the supply voltage Vdd. Conversely, the pMOS transistors P(i) of the first circuital branch 610 have to be biased in such a way as to electrically insulate the first input terminal INA from the output terminal OUT. Since the first input terminal INA is biased to the ground voltage GND, the first circuital branch 610 is subjected to a voltage difference (from the terminal connected to the output terminal OUT to the first input terminal INA) equal to the second input voltage mVdd. However, in order to use LV transistors only, each pMOS transistor P(i) of the second circuital branch 610 shall experience voltage differences across its control terminal and the other terminals thereof that are at most equal the value of the supply voltage Vdd. Thus, each pMOS transistor P(i) is biased in such a way that the total voltage drop between the output terminal OUT and the first input terminal INA (equal to the second input voltage mVdd) is equally distributed across all the pMOS transistors P(i) of the circuital branch. Since the number of pMOS transistors P(i) included in the first circuital branch 610 is equal to the (absolute value of the) second integer m, biasing the transistors' control terminals to voltages starting from (m+1)Vdd for the pMOS transistor P(m) (connected to the output terminal OUT), and proceeding to voltages higher and higher for the preceding pMOS transistors P(i) in the branch (for example, by rising the value of Vdd for each transistor), it is possible to ensure that each pMOS transistor P(i) is subjected to a voltage difference at most equal to the supply voltage Vdd, as desired. With this biasing scheme, the pMOS transistor P(1) (connected to the first input terminal INA) is surely turned off, having the control terminal at a voltage equal to that of the first input terminal INA *(i.e.,* the ground voltage GND).

The voltage switch 600 further includes additional circuital branches 630 (each one equal to the second circuital branch 620) connected to the first terminals of all the pMOS transistors P(i) of the first circuital branch 610, except for the pMOS transistor P(1). The purpose of said additional circuital branches 630 is to forcedly bias the first terminals of pMOS transistors P(i) of the first circuital branch 610 to a voltage equal to the voltages of the corresponding control terminals when the output terminal OUT is coupled with the second input terminal INB. In this way, the pMOS transistors P(i) of the first circuital branch 610 act as reverse-biased diodes. Since the nMOS transistors forming the additional circuital branches 630 are LV transistors, their control terminals have to be properly biased, in a way similar to that used for biasing the nMOS transistors N(j) of the second circuital branch 620. If said additional circuital branches 630 were absent, the first terminals of all the pMOS transistors P(i) would be floating, and their voltages might assume incorrect values, possibly causing the gate insulators to break or the PN junctions to breakdown. When the output terminal OUT has to be coupled with the first input terminal INA, the additional circuital branches 630 have to be properly deactivated.

According to a further embodiment of the invention, the possibility of using so-called Medium Voltage transistors (hereinafter, MV transistors) is contemplated. For the purposes of the present description, an MV transistor is a device designed in such a way to guarantee the capability of sustaining, at least between a pair of its terminals, voltage differences up-limited by a predetermined maximum voltage difference ΔVmm higher than the predetermined maximum voltage difference ΔVml that the LV transistors are guaranteed to sustain, but still lower than the predetermined maximum voltage difference ΔVmh guaranteed by HV transistors (in the example herein considered, an MV transistors is guaranteed to sustain, between two terminals thereof, a voltage difference higher than the supply voltage Vdd, but lower than the voltages to be handled by the voltage switch). Referring again to the example of voltage switches including MOS transistors, MV MOS transistors may have gate insulators of thickness capable to avoid insulator breaking with voltage differences Vm applied between the control terminal and the channel higher than the supply voltage Vdd, but lower than the maximum voltage difference that may occur between the output terminal OUT and the input terminals INA, INB (said maximum voltage difference is equal to the highest between the first and the second input voltages nVdd, mVdd). For example, if the voltage switches are integrated in a non-volatile memory IC, *e.g.*, a flash memory, an MV MOS transistor may have the same gate insulator thickness of a generic flash memory cell.

The possibility of including such MV devices in the circuital branches of the voltage switches allows to significantly simplify the structure thereof. More particularly, since, according to the embodiments of the present invention, the number of transistors to be included in a circuital branch can be obtained by dividing the (absolute value) difference between the voltage at the switch output terminal and the voltage at that, between the first and second input terminals, which is disconnected from the output terminal by the maximum voltage difference that a single transistor can sustain at its terminals (*i.e.,* Vdd = 3 Volts in case of LV transistors), said number of transistors can be drastically diminished, because an MV transistor can sustain voltage differences higher than the supply voltage Vdd.

Summarizing, the present invention provides a general pattern for realizing voltage switches adopting a particular circuital topology, that avoid the utilization of HV transistors. Assuming that:
- the voltage switch is implemented with transistors designed in such a way to guarantee the capability of sustaining voltage difference up-limited by a predetermined maximum voltage difference ΔVmx, for example equal to kVdd, *i.e.*, to the supply voltage Vdd multiplied by a predetermined multiplier coefficient k (the LV transistors mentioned in the present description belong to the case in which k is equal to one);
- the first input terminal is adapted to receive at least a first input voltage nVdd, equal to the supply voltage Vdd multiplied by a first integer n;
- the second input terminal is adapted to receive at least a second input voltage mVdd, equal to the supply voltage Vdd multiplied by a second integer m;
- the second input terminal receives the second input voltage mVdd when the first input terminal receives a third input voltage V3, (for example the ground voltage GND);
- the first input terminal receives the first input voltage nVdd when the second input terminal receives a fourth input voltage V4, (for example the ground voltage GND); and
- the first integer n is higher than the second integer m,
the voltage switch is such to include:
- a first circuital branch (connected between the first input terminal and the output terminal of the voltage switch) formed by a number of pMOS transistors equal to the smallest integer not less than the absolute value of ((mVdd-V3)/kVdd); and
- a second circuital branch (connected between the second input terminal and the output terminal of the voltage switch) formed by a number of nMOS transistors equal to the smallest integer not less than the absolute value of ((nVdd-V4)/(kVdd)).

When the first circuital branch is activated, a voltage drop equal to nVdd minus the fourth voltage V4 occurs between the output terminal and the second input terminal. Thus, the number of nMOS transistors forming the second circuital branch is sufficient to allow a distribution of said voltage drop along the second circuital branch such to up limit at the predetermined maximum voltage difference kVdd the voltage differences occurring between the control terminals and the other terminals of the latter transistors. In the same way, when a voltage drop equal to V3 minus mVdd occurs between the output terminal and the first input terminal *(i.e.,* when the second circuital branch is activated), the number of pMOS transistors forming the first circuital branch is sufficient to allow a distribution of said voltage drop along the first circuital branch such to up limit at the predetermined maximum voltage difference kVdd the voltage differences occurring between the control terminals and the other terminals of the latter transistors.

Mixed embodiment are possible, in which both LV transistors and MV transistors are included in at least one among the first and the second circuital branches of the voltage switches.

As previously described, the LV transistors are capable of sustaining voltage differences up-limited by a predetermined maximum voltage difference Δ Vml and the MV transistors are capable of sustaining voltage differences up-limited by a predetermined maximum voltage difference ΔVmm.

It is now assumed, for example, that both the first and the second circuital branches include both LV transistors and MV transistors. When the first circuital branch is activated, a voltage drop equal to nVdd minus the fourth voltage V4 occurs between the output terminal and the second input terminal. Said voltage drop can be properly distributed along the second circuital branch depending on the number of LV and MV transistors included in the second circuital branch. More particularly, if the second circuital branch includes a number L2 of LV transistors, capable altogether of sustaining a voltage drop of L2*ΔVml, the second circuital branch has to include a number M2 of MV transistors such as to sustain altogether the remaining voltage drop, given by nVdd minus the fourth voltage V4 minus L2*ΔVml. Consequently, the number M2 of nMOS MV transistors is equal to the smallest integer not less than the absolute value of ((nVdd-V4- L2*ΔVml)/(ΔVmm)).

In the same way, when the second circuital branch is activated, a voltage drop equal to mVdd minus the third voltageV3 occurs between the output terminal and the first input terminal. Again, said voltage drop can be properly distributed along the first circuital branch depending on the number of LV and MV transistors included in the first circuital branch. More particularly, if the first circuital branch includes a number L1 of LV transistors, capable altogether of sustaining a voltage drop of L1*ΔVml, the second circuital branch has to include a number M1 of MV transistors such as to sustain altogether the remaining voltage drop, given by mVdd minus the third voltage V3 minus L1*ΔVml. Consequently, the number M1 of pMOS MV transistors is equal to the smallest integer not less than the absolute value of ((mVdd-V3- L1*ΔVml)/(ΔVmm)).

The voltage switches using the circuital topology described in this document are advantageously used in high voltage ICs, particularly in memory ICs, for managing the voltages needed by the memory ICs for performing various operations, such as read, program and erase operations.

## Claims

1. A circuit comprising:
a first input terminal (INA) to provide a first high voltage n times larger than a supply voltage Vdd when a second input terminal (INB) assumes a reference level, the second input to provide a second high voltage m times larger than Vdd when the first input terminal assumes the reference level, wherein the absolute value of at least one of n and m is greater than unity;
an output terminal (OUT);
the circuit being **characterised in that** it comprises:
a first plurality of low voltage transistors (104, 204, 304, 306, 404, 406, 504, 506, 508, 610) coupled in series from the first input terminal to the output terminal and a second plurality of low voltage transistors (106, 108, 206, 208, 210, 308, 310,408,410,412, 510, 512, 514, 516, 620) coupled in series from the second input terminal to the output terminal, each low voltage transistor of the first and second plurality rated to sustain a maximum voltage difference between terminals that is below at least one of the first and second input voltages;
a biasing means (102, 202, 302, 402, 502) coupled to a control terminal of each low voltage transistor of the first and second pluralities, wherein the biasing means is configured to alternately couple the output terminal with one of the first and second input terminals while electrically isolating the other input terminal by biasing each of the control terminals at levels to switch ones of the first and second plurality between a pass state and a reversed diode state without exceeding the maximum voltage difference rating of any low voltage transistor of the first and second pluralities.

2. The circuit as in claim 1,
wherein the first input terminal (INA) is to provide a positive high voltage, the second input terminal (INB) is to provide a negative high voltage,
wherein the first plurality of low voltage transistors comprises a plurality of p-channel IGFET with source terminals coupled in series with drain terminals and each of the second plurality of low voltage transistors comprises a plurality of n-channel IGFET with source terminals coupled in series with drain terminals, and
wherein the biasing means (102, 202, 302, 402, 502) is configured to bias each of the control terminals at a level to alternately switch the output terminal (OUT)between the first and second input terminals while a voltage difference between the control terminal and other terminals of each of the low voltage transistors is no greater than the supply voltage Vdd.

3. The circuit as in claim 2, wherein the biasing means (102, 202, 302, 402, 502) is configured to provide a bias voltage greater than the supply voltage Vdd to one or more of transistor of the first and second plurality.

4. The circuit as in claim 3, wherein the biasing means (302, 402, 502) comprises additional low voltage transistors (312, 314, 414, 416, 418, 518-532) configured to selectively couple a plurality of high voltages greater than the supply voltage Vdd, but less than at least one of the first high voltage or the second high voltage, to the control terminals of the first and second plurality of transistors.

5. The circuit as in claim 3, wherein the biasing means is coupled a charge pump (CPA, CPB) to generate bias voltages greater than Vdd.

6. The circuit as in claim 5, wherein the first input terminal and the biasing means are each coupled a same charge pump (CPA) to provide input voltages and bias voltages greater than the supply voltage Vdd.

7. The circuit as in claim 5, wherein the biasing means (102, 202, 302, 402, 502) is configured to synchronously apply the generated bias voltages to the first and second plurality of low voltage transistors.

8. The circuit as in claim 2, wherein the first input terminal is coupled to a first charge pump (CPA) to provide the positive high voltage and the second input terminal is coupled to a second charge pump (CPB) to provide the negative high voltage.

9. The circuit as in claim 2, wherein the number of IGFET transistors in the first plurality of low voltage transistors is at least equal to the smallest integer not less than m, and
wherein the number of IGFET transistors in the second plurality of low voltage transistors is at least equal to the smallest integer not less than n.

10. The circuit as in claim 1, wherein when the first input (INA) provides the first high voltage, the biasing means (102, 202, 302, 402, 502) is configured to couple the output terminal OUT with the first input terminal while electrically isolating the second input terminal (INB) by biasing each control terminal of the first plurality of low voltage transistors to a pass voltage level equal in magnitude to the supply voltage Vdd multiplied by a quantity equal to one minus the number of transistors in the second plurality, and biasing, to the pass voltage, the control terminal of the low voltage transistor in the second plurality having a terminal directly coupled to the output terminal while biasing each successive serial transistor of the second plurality to a voltage reduced by the supply voltage Vdd from that of preceding transistor such that a last transistor of the second plurality coupled to the second input terminal is turned off.

11. The circuit as in claim 10, wherein when the second input (INB) provides the second high voltage, the biasing means (102, 202, 302, 402, 502) is configured to couple the output terminal with the second input terminal while electrically isolating the first input terminal (INA) by biasing each control terminal of the second plurality of low voltage transistors to a pass voltage level equal in magnitude to the supply voltage Vdd multiplied by a quantity equal to one minus the number of transistors in the first plurality, and biasing, to the pass voltage, the control terminal of the low voltage transistor in the first plurality having a terminal directly coupled to the output terminal while biasing each successive serial transistor of the first plurality to a voltage increased by Vdd from that of preceding transistor such that a last transistor of the first plurality coupled to the first input terminal is turned off.

12. The circuit as in claim 10, further comprising a third plurality of low voltage transistors (312, 314, 414, 416, 418, 518-532, 630), wherein ones of the third plurality are configured in series across a node between a first and second transistor of the first plurality and the biasing means, the biasing means further coupled to each control terminal of the third plurality to forcedly bias at least a terminal of the first transistor of the first plurality to a voltage equal to the voltages of the corresponding control terminals when the output terminal is coupled with the second input terminal.

13. The circuit as in claim 12, wherein the third plurality of low voltage transistors comprises the same number of transistors and of same channel conductivity as provided in the second plurality.

14. The circuit as in claim 13, wherein when the first input provides the first high voltage, the biasing means is configured to further bias, to the pass voltage, the control gate of the transistor of the third plurality that is directly coupled to a terminal of the first transistor of the first plurality and bias the control terminal of each successive serial transistor of the third plurality to a voltage decreased by Vdd from that of preceding transistor of the third plurality such that a last serial transistor of the third plurality having both a control terminal and a second terminal coupled to the biasing means is turned off.

15. The circuit as in claim 14, wherein when the second input provides the second high voltage, the biasing means is configured to further bias the control gate of each transistor of the third plurality such that each transistor of the third plurality is turned on while the last serial transistor of the third plurality having a second terminal coupled to the biasing means is biased to the pass voltage.

16. The circuit as in claim 15, wherein the bias level provided to the control gate of each transistor of the third plurality is equal to the bias level provided to the control gate of the second transistor of the first plurality.

17. An integrated circuit comprising:
at least a first, a second and a third (INA, INB, OUT) terminal,
a voltage switch circuit (100, 200, 300, 400, 500, 600) for selectively coupling the first terminal with the third terminal so as to transfer a voltage at the first terminal to the third terminals, and for selectively coupling the second terminal with the third terminal so as to transfer a voltage of the second terminal to the third terminal, **characterized in that**
the voltage switch circuit is the circuit of any one of the preceding claims.

18. The integrated circuit of claim 17, wherein the integrated circuit is a memory circuit.

## Patentansprüche

1. Schaltung, die Folgendes umfasst:
einen ersten Eingangsanschluss (INA) zum Bereitstellen einer ersten hohen Spannung, die n Mal größer ist als eine Speisespannung Vdd, wenn ein zweiter Eingangsanschluss (INB) einen Referenzpegel annimmt, wobei der zweite Eingang eine zweite hohe Spannung bereitstellt, die m Mal größer ist als Vdd, wenn der erste Eingangsanschluss den Referenzpegel annimmt, wobei der Absolutwert von n und/oder m größer als eins ist;
einen Ausgangsanschluss (OUT);
wobei die Schaltung **dadurch gekennzeichnet ist, dass** sie Folgendes umfasst:
eine erste Mehrzahl von Niederspannungstransistoren (104, 204, 304, 306, 404, 406, 504, 506, 508, 610), die in Serie vom ersten Eingangsanschluss zum Ausgangsanschluss gekoppelt sind, und eine zweite Mehrzahl von Niederspannungstransistoren (106, 108, 206, 208, 210, 308, 310, 408, 410, 412, 510, 512, 514, 516, 620), die in Serie vom zweiten Eingangsanschluss zum Ausgangsanschluss gekoppelt sind, wobei jeder Niederspannungstransistor der ersten und der zweiten Mehrzahl so bemessen ist, dass er eine maximale Spannungsdifferenz zwischen Anschlüssen aushält, die unter der ersten und/oder der zweiten Eingangsspannung liegt;
ein Vorspannmittel (102, 202, 302, 402, 502), das mit einem Steueranschluss jedes Niederspannungstransistors der ersten und der zweiten Mehrzahl gekoppelt ist, wobei das Vorspannmittel so konfiguriert ist, dass es den Ausgangsanschluss abwechselnd mit einem der ersten und zweiten Eingangsanschlüsse koppelt, während es den anderen Eingangsanschluss elektrisch isoliert, indem es jeden der Steueranschlüsse auf solche Pegel vorspannt, dass Transistoren der ersten und der zweiten Mehrzahl zwischen einem Durchlasszustand und einem Reverse-Diodenzustand geschaltet werden, ohne die maximale bemessene Spannungsdifferenz irgendeines Niederspannungstransistors der ersten und der zweiten Mehrzahl zu überschreiten.

2. Schaltung nach Anspruch 1,
wobei der erste Eingangsanschluss (INA) eine positive hohe Spannung bereitstellt, der zweite Eingangsanschluss (INB) eine negative hohe Spannung bereitstellt,
wobei die erste Mehrzahl von Niederspannungstransistoren eine Mehrzahl von p-Kanal-IGFET umfasst, wobei Source-Anschlüsse in Serie mit Drain-Anschlüssen gekoppelt sind und jeder aus der zweiten Mehrzahl von Niederspannungstransistoren eine Mehrzahl von n-Kanal-IGFET umfasst, wobei Source-Anschlüsse in Serie mit Drain-Anschlüssen gekoppelt sind, und
wobei das Vorspannmittel (102, 202, 302, 402, 502) so konfiguriert ist, dass es jeden der Steueranschlüsse auf einen solchen Pegel vorspannt, dass der Ausgangsanschluss (OUT) abwechselnd zwischen dem ersten und dem zweiten Eingangsanschluss geschaltet wird, während eine Spannungsdifferenz zwischen dem Steueranschluss und anderen Anschlüssen jedes der Niederspannungstransistoren nicht größer als die Speisespannung Vdd ist.

3. Schaltung nach Anspruch 2, wobei das Vorspannmittel (102, 202, 302, 402, 502) so konfiguriert ist, dass es eine Vorspannung, die größer ist als die Speisespannung Vdd, an einem oder mehreren Transistoren der ersten und der zweiten Mehrzahl bereitstellt.

4. Schaltung nach Anspruch 3, wobei das Vorspannmittel (302, 402, 502) zusätzliche Niederspannungstransistoren (312, 314, 414, 416, 418, 518-532) umfasst, die so konfiguriert sind, dass sie eine Mehrzahl von hohen Spannungen, die höher als die Speisespannung Vdd, aber niedriger als die erste hohe Spannung und/oder die zweite hohe Spannung sind, selektiv mit den Steueranschlüssen der ersten und zweiten Mehrzahl von Transistoren koppeln.

5. Schaltung nach Anspruch 3, wobei das Vorspannmittel mit einer Ladungspumpe (CPA, CPB) gekoppelt ist, um Vorspannungen zu erzeugen, die größer als Vdd sind.

6. Schaltung nach Anspruch 5, wobei der erste Eingangsanschluss und das Vorspannmittel jeweils mit derselben Ladungspumpe (CPA) gekoppelt sind, um Eingangsspannungen und Vorspannungen bereitzustellen, die größer sind als die Speisespannung Vdd.

7. Schaltung nach Anspruch 5, wobei das Vorspannmittel (102, 202, 302, 402, 502) so konfiguriert ist, dass es die erzeugten Vorspannungen synchron an die erste und die zweite Mehrzahl von Niederspannungstransistoren anlegt.

8. Schaltung nach Anspruch 2, wobei der erste Eingangsanschluss mit einer ersten Ladungspumpe (CPA) gekoppelt ist, um die positive hohe Spannung bereitzustellen, und der zweite Eingangsanschluss mit einer zweiten Ladungspumpe (CPB) gekoppelt ist, um die negative hohe Spannung bereitzustellen.

9. Schaltung nach Anspruch 2, wobei die Anzahl von IGFET-Transistoren in der ersten Mehrzahl von Niederspannungstransistoren wenigstens gleich der kleinsten ganzen Zahl nicht kleiner als m ist, und
wobei die Zahl der IGFET-Transistoren in der zweiten Mehrzahl von Niederspannungstransistoren wenigstens gleich der kleinsten ganzen Zahl nicht kleiner als n ist.

10. Schaltung nach Anspruch 1, wobei, wenn der erste Eingang (INA) die erste hohe Spannung bereitstellt, das Vorspannmittel (102, 202, 302, 402, 502) so konfiguriert ist, dass es den Ausgangsanschluss OUT mit dem ersten Eingangsanschluss koppelt, während es den zweiten Eingangsanschluss (INB) elektrisch isoliert, indem es jeden Steueranschluss der ersten Mehrzahl von Niederspannungstransistoren auf einen Durchlassspannungspegel vorspannt, dessen Größe gleich der Speisespannung Vdd multipliziert mit einer Größe von gleich eins minus der Anzahl der Transistoren in der zweiten Mehrzahl ist, und den Steueranschluss des Niederspannungstransistors in der zweiten Mehrzahl mit einem Anschluss auf die Durchlassspannung vorspannt, der direkt mit dem Ausgangsanschluss gekoppelt ist, und dabei jeden nachfolgenden seriellen Transistor der zweiten Mehrzahl auf eine Spannung vorspannt, die um die Speisespannung Vdd von der des vorhergehenden Transistors reduziert ist, so dass ein letzter Transistor der mit dem zweiten Eingangsanschluss gekoppelten zweiten Mehrzahl abgeschaltet wird.

11. Schaltung nach Anspruch 10, wobei, wenn der zweite Eingang (INB) die zweite hohe Spannung bereitstellt, das Vorspannmittel (102, 202, 302, 402, 502) so konfiguriert ist, dass es den Ausgangsanschluss mit dem zweiten Eingangsanschluss koppelt, während es den ersten Eingangsanschluss (INA) elektrisch isoliert, indem es jeden Steueranschluss der zweiten Mehrzahl von Niederspannungstransistoren auf einen Durchlassspannungspegel vorspannt, dessen Größe gleich der Speisespannung (Vdd) multipliziert mit einer Größe ist, die gleich eins minus der Anzahl der Transistoren in der ersten Mehrzahl ist, und den Steueranschluss des Niederspannungstransistors in der ersten Mehrzahl mit einem Anschluss auf die Durchlassspannung vorspannt, der direkt mit dem Ausgangsanschluss gekoppelt ist, und dabei jeden sukzessiven seriellen Transistor der ersten Mehrzahl auf eine Spannung vorspannt, die um Vdd von der des vorhergehenden Transistors erhöht ist, so dass ein letzter Transistor der mit dem ersten Eingangsanschluss gekoppelten ersten Mehrzahl abgeschaltet wird.

12. Schaltung nach Anspruch 10, die ferner eine dritte Mehrzahl von Niederspannungstransistoren (312, 314, 414, 416, 418, 518-532, 630) umfasst, wobei solche aus der dritten Mehrzahl in Serie über einen Knoten zwischen einem ersten und einem zweiten Transistor der ersten Mehrzahl und das Vorspannmittel konfiguriert sind, wobei das Vorspannmittel ferner mit jedem Steueranschluss der dritten Mehrzahl gekoppelt ist, um wenigstens einen Anschluss des ersten Transistors der ersten Mehrzahl zwangsweise auf eine Spannung vorzuspannen, die gleich den Spannungen der entsprechenden Steueranschlüsse ist, wenn der Ausgangsanschluss mit dem zweiten Eingangsanschluss gekoppelt wird.

13. Schaltung nach Anspruch 12, wobei die dritte Mehrzahl von Niederspannungstransistoren dieselbe Anzahl an Transistoren und dieselbe Kanalleitfähigkeit wie in der zweiten Mehrzahl bereitgestellt umfasst.

14. Schaltung nach Anspruch 13, wobei, wenn der erste Eingang die erste hohe Spannung bereitstellt, das Vorspannmittel so konfiguriert ist, dass es ferner das Steuergatter des Transistors der dritten Mehrzahl auf die Durchlassspannung vorspannt, der direkt mit einem Anschluss des ersten Transistors der ersten Mehrzahl gekoppelt ist, und den Steueranschluss jedes nachfolgenden seriellen Transistors der dritten Mehrzahl auf eine Spannung vorspannt, die um Vdd von der des vorhergehenden Transistors der dritten Mehrzahl verringert ist, so dass ein letzter serieller Transistor der dritten Mehrzahl, von dem ein Steueranschluss und ein zweiter Anschluss mit dem Vorspannmittel gekoppelt sind, abgeschaltet wird.

15. Schaltung nach Anspruch 14, wobei, wenn der zweite Eingang die zweite hohe Spannung bereitstellt, das Vorspannmittel so konfiguriert ist, dass es das Steuergatter jedes Transistors der dritten Mehrzahl weiter vorspannt, so dass jeder Transistor der dritten Mehrzahl eingeschaltet wird, während der letzte serielle Transistor der dritten Mehrzahl mit einem mit dem Vorspannmittel gekoppelten zweiten Anschluss auf die Durchlassspannung vorgespannt wird.

16. Schaltung nach Anspruch 15, wobei der an das Steuergatter jedes Transistors der dritten Mehrzahl angelegte Vorspannpegel gleich dem Vorspannpegel ist, der an das Steuergatter des zweiten Transistors der ersten Mehrzahl angelegt wird.

17. Integrierte Schaltung, die Folgendes umfasst:
wenigstens einen ersten, einen zweiten und einen dritten (INA, INB, OUT) Anschluss,
eine Spannungsumschalt-Schaltung (100, 200, 300, 400, 500, 600) zum selektiven Koppeln des ersten Anschlusses mit dem dritten Anschluss, um eine Spannung am ersten Anschluss zum dritten Anschluss zu übertragen und um den zweiten Anschluss selektiv mit dem dritten Anschluss zu koppeln, um eine Spannung des zweiten Anschlusses auf den dritten Anschluss zu übertragen, **dadurch gekennzeichnet, dass** die Spannungsumschalt-Schaltung die Schaltung nach einem der vorherigen Ansprüche ist.

18. Integrierte Schaltung nach Anspruch 17, wobei die integrierte Schaltung eine Speicherschaltung ist.

## Revendications

1. Un circuit comprenant:
une première borne d'entrée (INA) destinée à fournir une première haute tension n fois plus grande qu'une tension d'alimentation Vdd lorsqu'une deuxième borne d'entrée (INB) adopte une niveau de référence, la deuxième entrée étant destinée à fournir une deuxième haute tension m fois plus grande que la tension Vdd lorsque la première borne d'entrée adopte le niveau de référence, où la valeur absolue d'au moins une valeur parmi n et m est supérieure à l'unité,
une borne de sortie (OUT),
le circuit étant **caractérisé en ce qu'**il comprend :
une première pluralité de transistors basse tension (104, 204, 304, 306, 404, 406, 504, 506, 508, 610), couplés en série de la première borne d'entrée à la borne de sortie et une deuxième pluralité de transistors basse tension (106, 108, 206, 208, 210, 308, 310, 408, 410, 412, 510, 512, 514, 516, 620), couplés en série de la deuxième borne d'entrée à la borne de sortie, chaque transistor basse tension des première et deuxième pluralités étant réglé pour maintenir une différence de tension maximale entre bornes qui est inférieure à au moins une tension parmi les première et deuxième tensions d'entrée,
un moyen de polarisation (102, 202, 302, 402, 502) couplé à une borne de commande de chaque transistor basse tension des première et deuxième pluralités, où le moyen de polarisation est configuré de façon à coupler de manière alternée la borne de sortie avec une borne parmi la première et la deuxième borne d'entrée tout en isolant électriquement l'autre borne d'entrée en polarisant chacune des bornes de commande à des niveaux destinés à commuter des transistors des première et deuxième pluralités entre un état de passage et un état de diode inversée sans dépasser la valeur nominale de différence de tension maximale de tout transistor basse tension des première et deuxième pluralités.

2. Le circuit selon la Revendication 1,
où la première borne d'entrée (INA) est destinée à fournir une haute tension positive, la deuxième borne d'entrée (INB) est destinée à fournir une haute tension négative,
où la première pluralité de transistors basse tension comprend une pluralité de IGFET à canal p avec des bornes de source couplées en série à des bornes de drain et où chacun des transistors de la deuxième pluralité de transistors basse tension comprend une pluralité de IGFET à canal n avec des bornes de source couplées en série à des bornes de drain, et
où le moyen de polarisation (102, 202, 302, 402, 502) est configuré de façon à polariser chacune des bornes de commande à un niveau de façon à commuter de manière alternée la borne de sortie (OUT) entre les première et deuxième bornes d'entrée tandis qu'une différence de tension entre la borne de commande et les autres bornes de chacun des transistors basse tension n'est pas plus élevée que la tension d'alimentation Vdd.

3. Le circuit selon la Revendication 2, où le moyen de polarisation (102, 202, 302, 402, 502) est configuré de façon à fournir une tension de polarisation supérieure à la tension d'alimentation Vdd à un ou plusieurs des transistors des première et deuxième pluralités.

4. Le circuit selon la Revendication 3, où le moyen de polarisation (302, 402, 502) comprend des transistors basse tension additionnels (312, 314, 414, 416, 418, 518-532) configurés de façon à coupler de manière sélective une pluralité de hautes tensions supérieures à la tension d'alimentation Vdd mais inférieures à au moins une tension parmi la première haute tension ou la deuxième haute tension, aux bornes de commande des première et deuxième pluralités de transistors.

5. Le circuit selon la Revendication 3, où le moyen de polarisation est couplé à une pompe de charge (CPA, CPB) destinée à générer des tensions de polarisation supérieures à Vdd.

6. Le circuit selon la Revendication 5, où la première borne d'entrée et la moyen de polarisation sont chacun couplés à la même pompe de charge (CPA) de façon à fournir des tensions d'entrée et des tensions de polarisation supérieures à la tension d'alimentation Vdd.

7. Le circuit selon la Revendication 5, où le moyen de polarisation (102, 202, 302, 402, 502) est configuré de façon à appliquer de manière synchronisée les tensions de polarisation générées aux première et deuxième pluralités de transistors basse tension.

8. Le circuit selon la Revendication 2, où la première borne d'entrée est couplée à une première pompe de charge (CPA) de façon à fournir la haute tension positive et la deuxième borne d'entrée est couplée à une deuxième pompe de charge (CPB) de façon à fournir la haute tension négative.

9. Le circuit selon la Revendication 2, où le nombre de transistors IGFET dans la première pluralité de transistors basse tension est au moins égal au plus petit entier non inférieur à m, et
où le nombre de transistors IGFET dans la deuxième pluralité de transistors basse tension est au moins égal au plus petit entier non inférieur à n.

10. Le circuit selon la Revendication 1, où, lorsque la première entrée (INA) fournit la première haute tension, le moyen de polarisation (102, 202, 302, 402, 502) est configuré de façon à coupler la borne de sortie OUT à la première borne d'entrée tout en isolant électriquement la deuxième borne d'entrée (INB) en polarisant chacune des bornes de commande de la première pluralité de transistors basse tension sur un niveau de tension de passage égal en grandeur à la tension d'alimentation Vdd multipliée par une quantité égale à un moins le nombre de transistors de la deuxième pluralité et en polarisant, sur la tension de passage, la borne de commande du transistor basse tension de la deuxième pluralité possédant une borne directement couplée à la borne de sortie tout en polarisant chaque transistor en série successif de la deuxième pluralité sur une tension diminuée de la tension d'alimentation Vdd par rapport à celle du transistor précédent de sorte qu'un dernier transistor de la deuxième pluralité couplé à la deuxième borne d'entrée soit désactivé.

11. Le circuit selon la Revendication 10, où, lorsque la deuxième entrée (INB) fournit la deuxième haute tension, le moyen de polarisation (102, 202, 302, 402, 502) est configuré de façon à coupler la borne de sortie à la deuxième borne d'entrée tout en isolant électriquement la première borne d'entrée (INA) en polarisant chacune des bornes de commande de la deuxième pluralité de transistors basse tension sur un niveau de tension de passage égal en grandeur à la tension d'alimentation Vdd multipliée par une quantité égale à un moins le nombre de transistors de la première pluralité et en polarisant, sur la tension de passage, la borne de commande du transistor basse tension de la première pluralité possédant une borne directement couplée à la borne de sortie tout en polarisant chaque transistor en série successif de la première pluralité sur une tension augmentée de Vdd par rapport à celle du transistor précédent de sorte qu'un dernier transistor de la première pluralité couplé à la première borne d'entrée soit désactivé.

12. Le circuit selon la Revendication 10, comprenant en outre une troisième pluralité de transistors basse tension (312, 314, 414, 416, 418, 518-532, 630) où des transistors de la troisième pluralité sont configurés en série sur un noeud entre un premier et un deuxième transistors de la première pluralité et le moyen de polarisation, le moyen de polarisation étant couplé en outre à chaque borne de commande de la troisième pluralité de façon à polariser de force au moins une borne du premier transistor de la première pluralité sur une tension égale aux tensions des bornes de commande correspondantes lorsque la borne de sortie est couplée à la deuxième borne d'entrée.

13. Le circuit selon la Revendication 12, où la troisième pluralité de transistors basse tension comprend le même nombre de transistors et des transistors de la même conductivité de canal que ceux fournis dans la deuxième pluralité.

14. Le circuit selon la Revendication 13, où, lorsque la première entrée fournit la première haute tension, le moyen de polarisation est configuré de façon à polariser en outre, sur la tension de passage, la grille de commande du transistor de la troisième pluralité qui est directement couplée à une borne du premier transistor de la première pluralité et à polariser la borne de commande de chaque transistor en série successif de la troisième pluralité sur une tension diminuée de Vdd par rapport à celle du transistor précédent de la troisième pluralité de sorte qu'un dernier transistor en série de la troisième pluralité possédant à la fois une borne de commande et une deuxième borne couplée au moyen de polarisation soit désactivé.

15. Le circuit selon la Revendication 14, où, lorsque la deuxième entrée fournit la deuxième haute tension, le moyen de polarisation est configuré de façon à polariser en outre la grille de commande de chaque transistor de la troisième pluralité de sorte que chaque transistor de la troisième pluralité soit activé tandis que le dernier transistor en série de la troisième pluralité possédant une deuxième borne couplée au moyen de polarisation est polarisé sur la tension de passage.

16. Le circuit selon la Revendication 15, où le niveau de polarisation fourni à la grille de commande de chaque transistor de la troisième pluralité est égal au niveau de polarisation fourni à la grille de commande du deuxième transistor de la première pluralité.

17. Un circuit intégré comprenant :
au moins une première, une deuxième et une troisième borne (INA, INB, OUT),
un circuit de commutation de tension (100, 200, 300, 400, 500, 600) destiné à coupler de manière sélective la première borne avec la troisième borne de façon à transférer une tension de la première borne à la troisième borne et destiné à coupler de manière sélective la deuxième borne avec la troisième borne de façon à transférer une tension de la deuxième borne à la troisième borne, **caractérisé en ce que**
le circuit de commutation de tension est le circuit selon l'une quelconque des Revendications précédentes.

18. Le circuit intégré selon la Revendication 17, où le circuit intégré est un circuit de mémoire.
